(19) **Europäisches Patentamt**
**European Patent Office**
**Office européen des brevets**

(11) **EP 4 372 710 A1**

(12) # EUROPEAN PATENT APPLICATION
published in accordance with Art. 153(4) EPC

(43) Date of publication:
**22.05.2024 Bulletin 2024/21**

(21) Application number: **23734888.3**

(22) Date of filing: **29.06.2023**

(51) International Patent Classification (IPC):
*G08B 21/04* $^{(2006.01)}$   *G01P 15/00* $^{(2006.01)}$
*G01L 19/08* $^{(2006.01)}$   *G01D 21/02* $^{(2006.01)}$
*G06N 20/00* $^{(2019.01)}$

(86) International application number:
**PCT/KR2023/009120**

(87) International publication number:
**WO 2024/075935 (11.04.2024 Gazette 2024/15)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.10.2022 KR 20220129024**
**31.10.2022 KR 20220143186**

(71) Applicant: **Samsung Electronics Co., Ltd.**
**Suwon-Si, Gyeonggi-do 16677 (KR)**

(72) Inventors:
• **KIM, Hyeonseong**
**Suwon-si, Gyeonggi-do 16677 (KR)**
• **PARK, Jeongmin**
**Suwon-si, Gyeonggi-do 16677 (KR)**

(74) Representative: **HGF**
**HGF Limited**
**1 City Walk**
**Leeds LS11 9DX (GB)**

(54) **ELECTRONIC DEVICE AND METHOD FOR PERFORMING FALL DETECTION**

(57)    According to an example, the electronic device includes at least one sensor, a memory, and a processor. The processor is configured to obtain first information related to a first walking pattern of a user of the electronic device and second information related to a walking environment of the user. The processor is configured to identify, based on the first information and third information related to a reference walking pattern which is stored in a memory, a first value indicating a difference between the first walking pattern and the reference walking pattern. The processor is configured to identify a second value indicating a difference between the first walking pattern and the second walking pattern. The processor is configured to identify a threshold value related to fall detection based on the first value, the second value, and the second information. The processor is configured to execute a function for the fall detection.

FIG. 2

**Description**

**[Technical Field]**

The following descriptions relate to an electronic device and a method for performing fall detection.

**[Background Art]**

As technology develops, various services are provided through an electronic device. The electronic device may identify a movement of a user carrying the electronic device by using at least one sensor. The electronic device may identify various activity states of the user based on the movement of the user. For example, the electronic device may identify information on the steps of the user. The electronic device may provide various services related to the steps of the user to the user.

The above-described information may be provided as related art for the purpose of assisting understanding of the present disclosure. No claim or decision is raised as to whether any of the above-described contents may be applied as a prior art related to the present disclosure.

**[Disclosure]**

**[Technical Solution]**

According to an example, the electronic device may comprise at least one sensor, a memory, and a processor. The processor may be configured to obtain, using the at least one sensor, first information related to a first walking pattern of a user of the electronic device and second information related to a walking environment of the user. The processor may be configured to identify, based on the first information and third information related to a reference walking pattern which is stored in the memory, a first value indicating a difference between the first walking pattern and the reference walking pattern. The processor may be configured to identify, based on the first information and fourth information related to a second walking pattern of the user, which is stored in the memory and identified based on a walking history of the user, a second value indicating a difference between the first walking pattern and the second walking pattern. The processor may be configured to identify, based on the first value, the second value, and the second information, a threshold value related to fall detection. The processor may be configured to execute, based on comparison between the threshold value and a value obtained through the at least one sensor, a function for the fall detection.

According to an example, a method of an electronic device may comprise obtaining, using at least one sensor of the electronic device, first information related to a first walking pattern of a user of the electronic device and second information related to a walking environment of the user, identifying, based on the first information and third information related to a reference walking pattern which is stored in a memory, a first value indicating a difference between the first walking pattern and the reference walking pattern, identifying, based on the first information and fourth information related to a second walking pattern of the user, which is stored in the memory and identified based on a walking history of the user, a second value indicating difference between the first walking pattern and the second walking pattern, identifying, based on the first value, the second value, and the second information, a threshold value related to fall detection, and executing, based on comparison between the threshold value and a value obtained through the at least one sensor, a function for the fall detection.

**[Description of the Drawings]**

FIG. 1 is a block diagram of an electronic device in a network environment according to an example.
FIG. 2 is a simplified block diagram of an electronic device according to an example.
FIG. 3 illustrates a simplified block diagram of a processor included in an electronic device according to an example.
FIG. 4 is a flowchart illustrating an operation of an electronic device according to an example.
FIG. 5 is a flowchart illustrating an operation of an electronic device according to an example.
FIGS. 6A to 6C illustrate examples of trends for identifying a walking environment of the user, according to an example.
FIGS. 7A to 7C illustrate examples of trends for identifying a walking environment of the user, according to an example.
FIG. 8 illustrates an example of a trend for identifying a walking environment of the user, according to an example.
FIG. 9 illustrates an example of an operation of an electronic device for learning data according to an example.
FIG. 10 is a flowchart illustrating an operation of an electronic device, according to an example.
FIG. 11 illustrates an example of an operation of an electronic device according to an example.

FIG. 12 illustrates an example of an operation of an electronic device according to an example.

FIG. 13 is a flowchart illustrating an operation of an electronic device, according to an example.

FIG. 14 illustrates an example of a designated matrix according to an example.

FIG. 15 illustrates an example of a matrix indicating a population according to an example.

FIG. 16 illustrates an example of a matrix indicating information on fitness according to an example.

FIG. 17 illustrates an example of a matrix indicating at least a part of a population according to an example.

FIG. 18 illustrates an example of a learning result for a walking pattern according to an example.

FIG. 19 illustrates an example of an operation mode of a processor according to an example.

FIGS. 20A and 20B illustrate an example in which values for a walking pattern according to an example are displayed as three-dimensional coordinates.

FIGS. 21A and 21B illustrate an example of a screen for warning of a fall hazard according to an example.

**[Mode for Invention]**

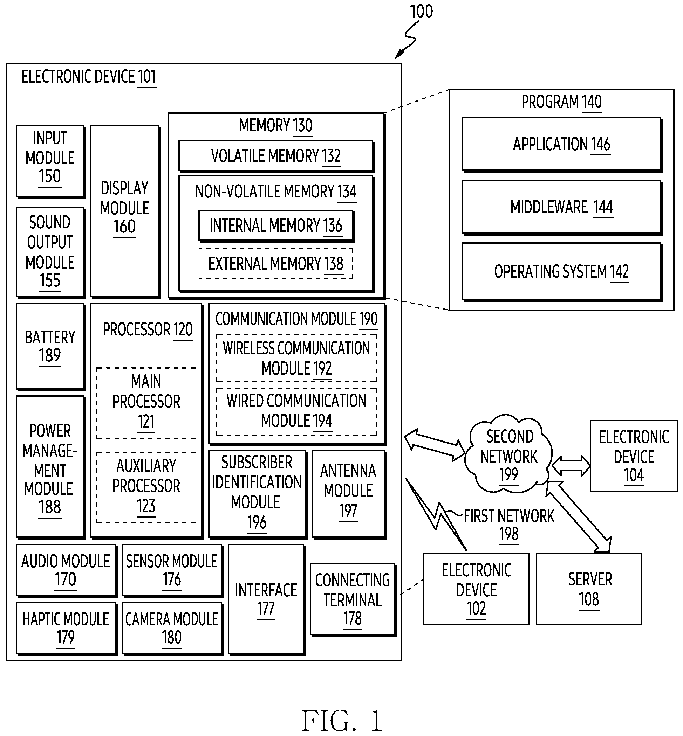

FIG. 1 is a block diagram illustrating an electronic device 101 in a network environment 100 according to various embodiments.

Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module(SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth™, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN))). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an example, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an example, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another example, the external electronic device 104 may include an internet-of things (IoT) device. The server 108 may be an intelligent server using machine learning and/or a neural network. According to an example, the external electronic device 104 or the server 108 may be included in the second network 199. The electronic device 101 may be applied to intelligent services (e.g., smart home, smart city, smart car, or healthcare) based on 5G communication technology or IoT-related technology.

According to an example, the electronic device (e.g., the electronic device 101 of FIG. 1) may provide a notification to the user based on identifying that a fall occurs to the user. The electronic device may learn a walking pattern of the user before identifying that a fall occurs. Based on the difference between the learned walking pattern and the walking pattern identified through the sensor, the electronic device may identify a threshold value related to fall detection to the user. The electronic device may execute a function for fall detection based on the identified threshold value.

In the following specification, an example for identifying the threshold value related to fall detection to the user based on the difference between the learned walking pattern and the walking pattern identified through the sensor and executing the function for fall detection based on the identified threshold value may be described.

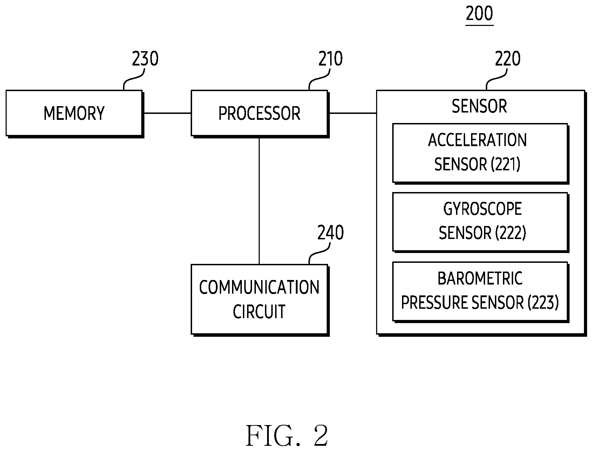

FIG. 2 is a simplified block diagram of an electronic device according to an example.

Referring to FIG. 2, an electronic device 200 may be configured in various forms. The electronic device 200 may be implemented in various forms that may be carried by the user, such as a smart phone, smart watch, smart band, smart ring, wireless earphone, or smart glasses. For example, the electronic device 200 may correspond to an electronic device 101 of FIG. 1.

According to an example, the electronic device 200 may include a processor 210, a sensor 220, a memory 230, and/or a communication circuit 240. According to an example, the electronic device 200 may include at least one of the processor 210, the sensor 220, the memory 230, and the communication circuit 240. For example, at least a part of the processor 210, the sensor 220, the memory 230, and the communication circuit 240 may be omitted according to an example.

According to an example, the processor 210 may correspond to the processor 120 of FIG. 1. The processor 210 may be operatively or operably coupled with or connected with the sensor 220, the memory 230, and the communication circuit 240. For example, that the processor 210 is operatively coupled with another component may mean that the processor 210 may control another component. For example, the processor 210 may control the operation of the sensor 220. The processor 210 may activate or deactivate the sensor 220. The processor 210 may process information obtained from the sensor 220.

According to an example, the processor 210 may include at least one processor. The processor 210 may include at least one processor. According to an example, the processor 210 may include a hardware component for processing data based on one or more instructions. The hardware component for processing data may include, for example, an arithmetic and logic unit (ALU), a field programmable gate array (FPGA), and/or a central processing unit (CPU).

According to an example, the electronic device 200 may include the sensor 220. The sensor 220 may include at least one sensor. For example, a sensor 430 may correspond to a sensor module 176 of FIG. 1. For example, the sensor 220 may include at least one of an acceleration sensor 221, a gyroscope sensor 222, and a barometric pressure sensor 223.

For example, the acceleration sensor 221 may identify (or measure or detect) the acceleration of the electronic device 200 in three directions of x-axis, y-axis, and z-axis. For example, the gyroscope sensor 222 may identify (or measure or detect) the angular velocity of the electronic device 200 in the three directions of the x-axis, the y-axis, and the z-axis. According to an example, the electronic device 200 may include an inertial sensor configured with the acceleration sensor 221 and the gyroscope sensor 222.

For example, the barometric pressure sensor 223 may identify (or measure or detect) the air pressure around the electronic device 200. The processor 210 may identify the height (or altitude) at which the electronic device 200 is located from the ground based on data on the air pressure around the electronic device 200 identified using the barometric pressure sensor 223.

Although not illustrated, the sensor 220 may further include a sensor for obtaining (or identifying, measuring, and detecting) various data about the user.

For example, the sensor 220 may include a body temperature sensor. The processor 210 may measure the skin temperature of a part of the body of the user through the body temperature sensor. The processor 210 may obtain the body of the user temperature based on the skin temperature of the part of the body of the user.

For example, the sensor 220 may include a heart rate variability (HRV) sensor. The processor 210 may measure heartbeat regularity or variability through the HRV sensor. The processor 210 may obtain information about the heartbeat regularity or variability through the HRV sensor.

According to an example, the electronic device 200 may include the memory 230. The memory 230 may be used to store information or data. For example, the memory 230 may be used to store data obtained from the user. For example, the memory 230 may correspond to the memory 130 of FIG. 1. For example, the memory 230 may be a volatile memory unit or units. For example, the memory 230 may be a non-volatile memory unit or units. For example, the memory 230 may be another form of computer readable medium, such as a magnetic or optical disk. For example, the memory 230 may store data obtained based on an operation (e.g., an algorithm execution operation) performed by the processor 210. For example, the memory 230 may store data (e.g., data related to the walking of the user) obtained by the sensor 220.

According to an example, the electronic device 200 may include a communication circuit 240. For example, the communication circuit 240 may correspond to a communication module 190 of FIG. 1.

For example, the communication circuit 240 may be used for various radio access technologies (RATs). For example, the communication circuit 240 may be used to perform Bluetooth communication, wireless local area network (WLAN) communication, or ultra wideband (UWB) communication. For example, the communication circuit 240 may be used to perform cellular communication.

According to an example, the electronic device 200 may include various components in addition to the illustrated components. For example, the electronic device 200 may include a display. The display may be used to display various screens. For example, the display may be used to output content, data, or signals through the screen. For example, the display may correspond to a display module 160 of FIG. 1. For example, the electronic device 200 may further include a global positioning system (GPS) circuit. The GPS circuit may be used to identify the location of the electronic device 200.

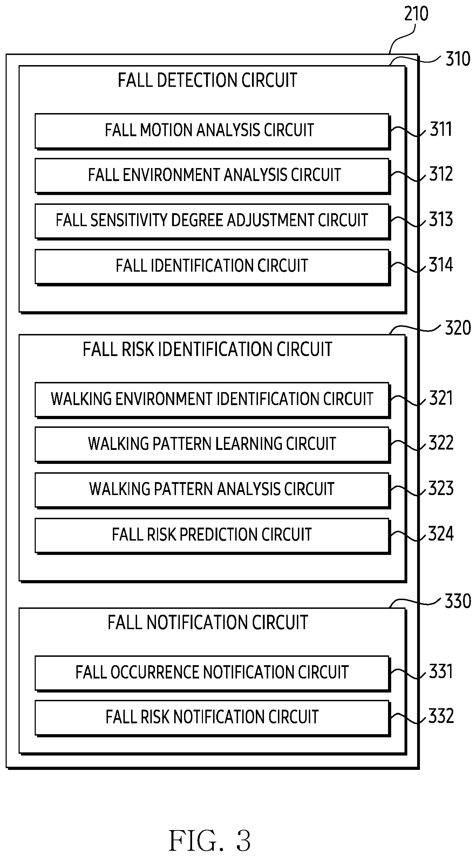

FIG. 3 illustrates a simplified block diagram of a processor included in an electronic device according to an example.

Referring to FIG. 3, the processor 210 may include at least one of a fall detection circuit 310, a fall hazard identification circuit 320, and a fall notification circuit 330. The processor 210 may monitor whether a fall occurs using the fall detection circuit 310. The processor 210 may predict the fall hazard of the user by monitoring the walking of the user and analyzing and learning the walking pattern of the user until the fall hazard is predicted by using the fall hazard identification circuit 320.

For example, the fall detection circuit 310 may include at least one of a fall motion analysis circuit 311, a fall environment analysis circuit 312, a fall sensitivity degree adjustment circuit 313, and a fall identification circuit 314. The processor 210 may identify a movement of the user when detecting a fall by using the fall motion analysis circuit 311 of the fall detection circuit 310. The processor 210 may identify the environment around the electronic device 200 (or the user) when detecting a fall by using the fall environment analysis circuit 312 of the fall detection circuit 310. The processor 210 may change the sensitivity degree for fall identification in case of a situation that possibility of the fall for the user is high, by using the fall sensitivity degree adjustment circuit 314 of the fall detection circuit 310. The processor 210 may identify that the fall has occurred to the user based on information on the movement of the user by using the fall identification circuit 314 of the fall detection circuit 310.

For example, the fall hazard identification circuit 320 may include at least one of a walking environment identification circuit 321, a walking pattern learning circuit 322, a walking pattern analysis circuit 323, and a fall hazard prediction circuit 324. The processor 210 may identify the environment around the electronic device 200 (or the user) when the user walks by using the walking environment identification circuit 321 of the fall hazard identification circuit 320. The processor 210 may continuously learn the walking pattern of the user when the user walks by using the walking pattern learning circuit 322 of the fall hazard identification circuit 320. The processor 210 may obtain and store information on the walking pattern of the user by using the walking pattern analysis circuit 323 of the fall hazard identification circuit 320. The processor 210 may identify the probability of a fall occurring to the user based on the walking environment and walking pattern by using the fall hazard prediction circuit 324 of the fall hazard identification circuit 320.

For example, the fall notification circuit 330 may include at least one of a fall occurrence notification circuit 331 and a fall hazard notification circuit 332. By using the fall occurrence notification circuit 331 of the fall notification circuit 330, when a fall occurs to the user, the processor 210 may provide the user or another user with a notification indicating that the fall has occurred to the user, and may transmit a rescue signal through an emergency contact. The processor 210 may provide the user with the notification indicating that the probability of the fall is high, in case that the probability of the fall occurring to the user is high, by using the fall hazard notification circuit 332 of the fall notification circuit 330.

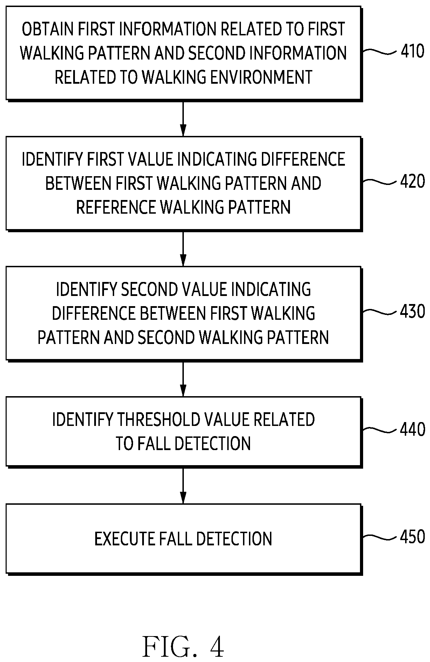

FIG. 4 is a flowchart illustrating an operation of an electronic device according to an example.

Referring to FIG. 4, in operation 410, the processor 210 may obtain first information related to a first walking pattern (e.g., a gait, a movement pattern etc. - the feature should not be understood as limited to walking but may also be a running pattern or other pattern of movement) and second information related to a walking environment (e.g., the surroundings of the electronic device 200, a geographic area in the vicinity of the electronic device 200, walking conditions etc.). For example, the processor 210 may obtain the first information related to the first walking pattern of the user of the electronic device 200 and the second information related to the walking environment of the user by using a sensor 220 (or at least one sensor) and/or a communication circuit 240.

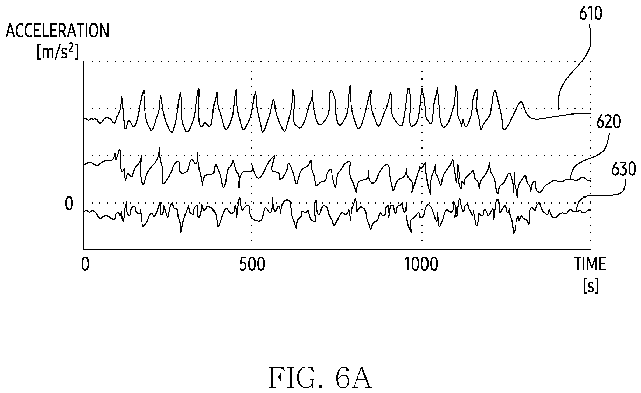

According to an example, the processor 210 may obtain first information related to the first walking pattern of the user of the electronic device 200 by using the sensor 220. For example, the processor 210 may obtain first information related to the first walking pattern based on first data obtained during the designated time interval (e.g., a first time period) by using the acceleration sensor 221. The processor 210 may obtain trends of acceleration corresponding to each of 3 axis directions, during the designated time interval. The processor 210 may identify the first walking pattern of the user based on difference of sliding window summing identified based on the obtained trends of acceleration. An operation in which the processor 210 identifies the first walking pattern of the user will be described later with reference to FIGS. 6A to 8.

According to an example, the processor 210 may obtain second information related to the walking environment of the user of the electronic device 200. For example, the processor 210 may obtain second information related to the walking environment of the user based on second data obtained by using at least one of the acceleration sensor 221, a gyroscope sensor 222, a barometric pressure sensor 223, and a communication circuit 240. For example, the second information may include information on a gradient of a moving path of the electronic device 200 (i.e., information on a gradient of a path on which the electronic device 200 is moving or travelling), information on a moving state of the electronic device 200, and information on weather during the designated time interval.

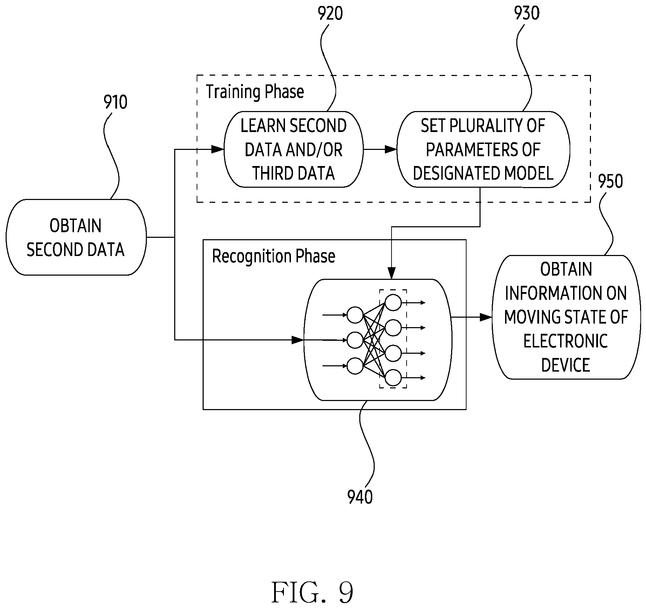

For example, the processor 210 may obtain information on the moving state of the electronic device 200 by setting at least a part of the second data obtained by using at least one of the acceleration sensor 221, the gyroscope sensor 222, and the barometric pressure sensor 223 as input data of a designated model (e.g., an artificial intelligence and/or machine learning model) indicated by a plurality of parameters. For example, the plurality of parameters of the designated model may be set based on learning of the third data identified according to the walking history of the user. An example in which the processor 210 obtains information on the moving state of the electronic device 200 by using the designated model will be described later with reference to FIG. 9.

For example, the processor 210 may obtain the information on weather during the designated time interval from an external electronic device (e.g., a server) by using the communication circuit 240. For example, the external electronic device may have knowledge of the weather in the vicinity of the electronic device 200. For example, the processor 210 may obtain the information on a gradient of a moving path from an external electronic device (e.g., a server) by using a communication circuit 240. For example, the external electronic device may store data regarding a topology of the environment or area in which the electronic device 200 is located, such that information on the gradient of a path on which the electronic device 200 is moving (e.g., information on the gradient of the ground in the direction of motion of the electronic device 200) may be obtained.

In operation 420, the processor 210 may identify a first value (or other indication) indicating a difference between the first walking pattern and a reference walking pattern. For example, the processor 210 may identify, based on the first information and third information related to a reference walking pattern which is stored in the memory 230, the first value indicating the difference between the first walking pattern and the reference walking pattern. For example, the processor 210 may identify (e.g., via comparison or computation) a first similarity between the first walking pattern and the reference walking pattern based on the first information and the third information. For example, the first value may be a scalar value, a vector, an array of values etc.

According to an example, the processor 210 may store the third information related to the reference walking pattern in the memory 230. For example, the reference walking pattern may mean a walking pattern of general users (e.g., a generalized or an averaged walking pattern associated with a plurality of users, a default walking pattern associated with typical users etc.). For example, the processor 210 may receive third information related to the reference walking pattern from the external electronic device (e.g., the server) by using the communication circuit 240. The processor 210 may store the received third information in the memory 230. The third information may be obtained based on walking patterns related to a plurality of users stored in the external electronic device (e.g., the server). For example, the third information related to the reference walking pattern may be a state stored in the memory 230 of the electronic device 200.

According to an example, the processor 210 may identify the first value indicating the difference between the first walking pattern and the reference walking pattern based on the first information and the third information. The processor 210 may identify one or more values related to the first walking pattern. The processor 210 may identify one or more values related to the reference walking pattern. For example, the reference walking pattern may correspond to the one or more values related to the first walking pattern, such as by relating to the same walking pattern features as those on which the one or more values related to the first walking pattern are identified on the basis of.

The processor 210 may identify the first value indicating the difference between the first walking pattern and the reference walking pattern based on a Euclidean distance between values related to the first walking pattern (e.g., representative value(s)) and values related to the reference walking pattern (e.g., representative value(s)). The processor 210 may identify the first similarity between the first walking pattern and the reference walking pattern based on the Euclidean distance between the values related to the first walking pattern and the values related to the reference walking pattern. For example, the first value and the first similarity indicating the difference between the first walking pattern and the reference walking pattern may be inversely proportional. The greater the first value indicating the difference between the first walking pattern and the reference walking pattern increases, the smaller the first similarity may be. The smaller the first value indicating the difference between the first walking pattern and the reference walking pattern, the greater the first similarity may be.

In operation 430, the processor 210 may identify a second value (or other indication) indicating a difference between the first walking pattern and a second walking pattern. For example, the processor 210 may identify, based on the first information and fourth information related to a second walking pattern of the user, which is stored in the memory 230 and identified based on the walking history of the user, the second value indicating the difference between the first walking pattern and the second walking pattern. For example, the processor 210 may identify a second similarity between the first walking pattern and the second walking pattern based on the first information and the fourth information. For example, the second value may be a scalar value, a vector, an array of values etc.

According to an example, the processor 210 may store the fourth information related to the second walking pattern in the memory 230. The processor 210 may identify the fourth information related to the second walking pattern based on the walking history of the user. For example, the processor 210 may perform learning of the walking pattern of the user based on the walking history of the user. The processor 210 may obtain fourth information related to the second walking pattern based on performing learning of the walking pattern of the user. The processor 210 may store the obtained fourth information in the memory 230. For example, the fourth information related to the second walking pattern may be the state stored in the memory 230 of the electronic device 200.

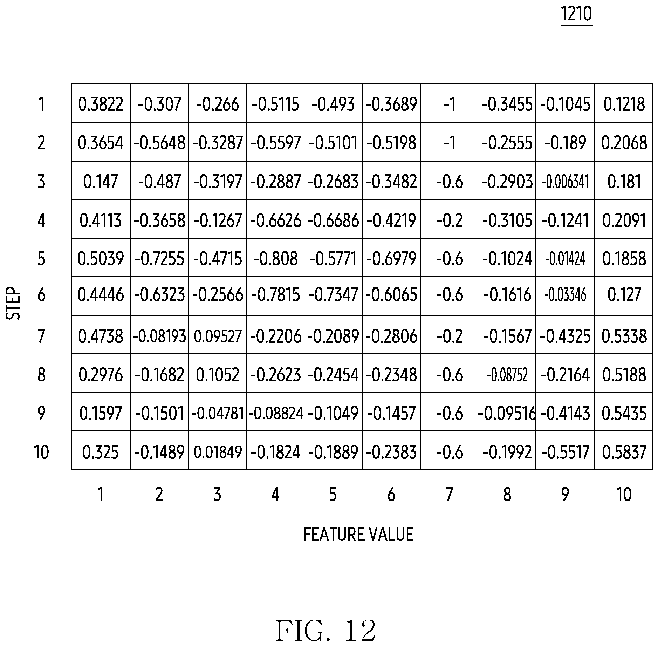

For example, the processor 210 may identify a population including values related to a walking pattern (e.g., a gait or other movement pattern) according to a plurality of steps of the user based on the walking history of the user. For example, the processor 210 may identify first steps of the user by using the sensor 220. The processor 210 may identify feature values (e.g. feature values are described later in relation to FIG. 12) according to the first steps based on identifying that the number of first steps is equal to or greater than a designated number of times. The processor 210 may identify feature values for second steps of the user that occur before the first steps are identified. The feature values for the second steps may be stored in the memory 230. For example, the feature values for the second steps may be values learned according to the walking history of the user.

The processor 210 may identify values related to the walking pattern according to the first steps based on the feature values according to the first steps. The processor 210 may identify values related to the walking pattern according to the second steps based on the feature values according to the second steps. The processor 210 may configure values related to the walking pattern according to the first steps and values related to the walking pattern according to the second steps as a population including values (or other information) related to the walking pattern according to the plurality of steps.

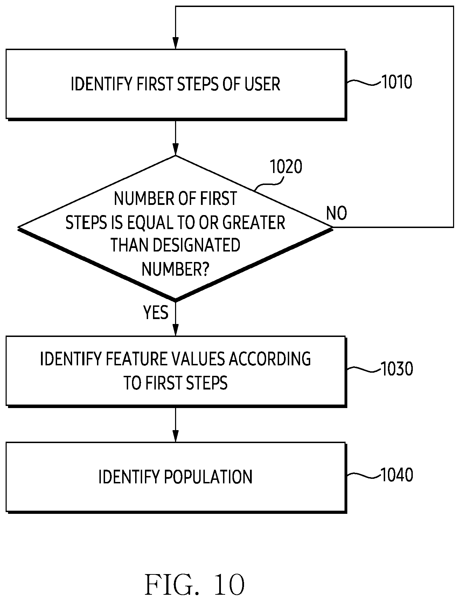

For example, the processor 210 may identify at least a part of the population according to information on fitness according to a plurality of steps obtained based on the identified population. The processor 210 may perform learning on the walking pattern of the user based on at least a part of the population. A specific example of an operation of performing learning on the walking pattern of the user will be described later with reference to FIGS. 10 to 18.

According to an example, the processor 210 may identify the second value indicating the difference between the first walking pattern and the second walking pattern based on the first information and the fourth information. The processor 210 may identify one or more values related to the first walking pattern. The processor 210 may identify one or more values related to the second walking pattern. For example, these may correspond to the one or more values related to the first walking pattern, such as by relating to the same walking pattern features as those on which the one or more values related to the first walking pattern are identified on the basis of. The processor 210 may identify the second value indicating the difference between the first walking pattern and the second walking pattern based on the Euclidean distance between the values related to the first walking pattern and the values related to the second walking pattern. The processor 210 may identify the second similarity between the first walking pattern and the second walking pattern based on the Euclidean distance between the values related to the first walking pattern and the values related to the second walking pattern. For example, the second value and the second similarity indicating the difference between the first walking pattern and the second walking pattern may be inversely proportional. The greater the second value indicating the difference between the first walking pattern and the second walking pattern, the smaller the second similarity may be. The smaller the second value indicating the difference between the first walking pattern and the second walking pattern, the greater the second similarity may be.

In operation 440, the processor 210 may identify a threshold value (e.g., a preset value, a predefined indication) related to fall detection. For example, the processor 210 may identify the threshold value related to the fall detection based on the first value, the second value, and the second information (or based on at least one of the first value, the second value, and the second information, such as based on the second value and the second information).

In operation 450, the processor 210 may execute a function for the fall detection. For example, the processor 210 may execute the function for the fall detection based on a comparison between the identified threshold value and the value obtained through the sensor 220.

For example, the threshold value related to the fall detection may include a reference value (or other indication) for determining whether the fall has occurred to the user. The value obtained through the sensor 220 may include a value related to an impact amount, a value related to an air pressure change amount, and/or a value related to the time when the activity of the user is ceased. For example, the processor 210 may identify that the fall has occurred to the user based on identifying that the value for determining the fall of the user is equal to or greater than the threshold value. For example, the threshold value related to the fall detection may include a first threshold value related to the impact amount, a second threshold value related to the air pressure change amount, and/or a third threshold value related to the activity cessation maintenance time (or activity cessation time, e.g., an amount of time for which an activity (such as movement, e.g., walking) is stopped or interrupted).

For example, the processor 210 may identify (or determine, assume, detect etc.) that the user has fallen based on identifying(or determining) that the value related to the impact amount obtained through the sensor 220 is equal to or greater than the first threshold value. For example, the processor 210 may identify that the fall has occurred to the user based on identifying that the value related to the air pressure change amount obtained through the sensor 220 is equal to or greater than the second threshold value. For example, processor 210 may identify that the fall has occurred to the user based on identifying that the time when the activity of the user is ceased is equal to or greater than the third threshold value.

For example, the processor 210 may identify that the user has had a fall based on identifying that the value related to the impact amount obtained through the sensor 220 is equal to or greater than the first threshold value, the value related to the air pressure change amount obtained through the sensor 220 is equal to or greater than the second threshold value, and the time when the activity of the user is ceased is equal to or greater than the third threshold value.

According to an example, the processor 210 may identify a probability value that the fall occurs based on the first value, the second value, and the second information. The processor 210 may identify the threshold value as one of a plurality of candidate threshold values based on the identified probability value. For example, the processor 210 may identify a fall hazard degree as one of 'low', 'normal', 'high', and 'very high' based on the probability value that the user has fallen. The processor 210 may identify one of the plurality of candidate threshold values according to the fall hazard degree. In a non-limiting example, the plurality of candidate threshold values according to the fall hazard degree may be set as shown in Table 1.

[Table 1]

| fall hazard degree | impact amount [G] | air pressure change amount [hPa] | activity cessation time [s] |
|---|---|---|---|
| low | 20 | 0.4 | 60 |
| normal | 20 | 0.4 | 60 |
| high | 15 | 0.32 | 40 |
| very high | 10 | 0.32 | 40 |

Referring to Table 1, the plurality of candidate threshold values may be set according to the fall hazard degree. For example, in case that the fall hazard degree is 'low', the first threshold value related to the impact amount may be set to 20 [G] (G-force). In case that the fall hazard degree is 'low', the second threshold value related to the air pressure change amount may be set to 0.4 [hPa] (hectopascal). In case that the fall hazard degree is 'low', the third threshold value related to the activity cessation time may be set to 60 [s] (second). For example, in case that the fall hazard degree is 'normal', the first threshold value related to the impact amount may be set to 20 [G]. In case that the fall hazard degree is 'normal', the second threshold value related to the air pressure change amount may be set to 0.4 [hPa]. In case that the fall hazard degree is 'normal', the third threshold value related to the activity cessation time may be set to 60 [s] (second). For example, in case that the fall hazard degree is 'high', the first threshold value related to the impact amount may be set to 15 [G]. In case that the fall hazard degree is 'high', the second threshold value related to the air pressure change amount may be set to 0.32 [hPa]. In case that the fall hazard degree is 'high', the third threshold value related to the activity cessation time may be set to 40 [s] (second). For example, in case that the fall hazard degree is 'very high', the first threshold value related to the impact amount may be set to 10 [G]. In case that the fall hazard degree is 'very high', the second threshold value related to the air pressure change amount t may be set to 0.32 [hPa]. In case that the fall hazard degree is 'very high', the third threshold value related to the activity cessation time may be set to 40 [s] (second).

According to an example, the processor 210 may identify the probability value that a fall will occur for the user and may identify the fall hazard degree based on the identified probability value. The processor 210 may change a sensitivity degree of the fall detection algorithm by changing the threshold value related to the fall detection according to the fall hazard degree. The processor 210 may quickly identify that the fall has occurred by changing the threshold value (e.g., the first threshold value, the second threshold value, and the third threshold value) related to the fall detection.

For example, the processor 210 may transmit a signal for causing the external electronic device to the external electronic device, in order to provide the notification for warning of the fall hazard through the external electronic device worn by the user and connected to the electronic device 200, based on the identified probability value and the second information.

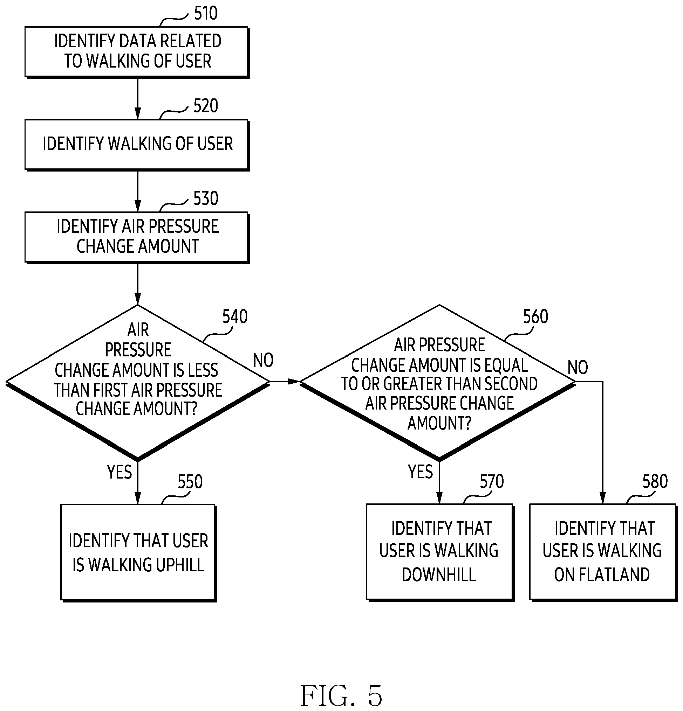

FIG. 5 is a flowchart illustrating an operation of an electronic device according to an example.

Referring to FIG. 5, in operation 510, a processor 210 may identify data related to walking (or other directional movement, such as running etc.) of a user. For example, the processor 210 may identify data related to acceleration of an electronic device 200 by using an acceleration sensor 221. For example, the processor 210 may identify data on related to the angular velocity of the electronic device 200 by using a gyroscope sensor 222. For example, the processor 210 may identify data related to air pressure (or altitude of the electronic device 200) by using a barometric pressure sensor 223.

In operation 520, the processor 210 may identify the walking (or other movement) of the user. For example, the processor 210 may identify the walking of the user based on data related to the walking of the user.

According to an example, the processor 210 may identify the steps of the user based on data related to the walking of the user. The processor 210 may identify the steps of the user based on data on the 3 axis acceleration. The processor 210 may identify the walking of the user based on identifying that the steps of the user are equal to or greater than a designated number of times.

In operation 530, the processor 210 may identify the air pressure change amount based on identifying the walking of the user. For example, the processor 210 may identify the air pressure change amount through the data identified by using the barometric pressure sensor 223, based on identifying the walking of the user. For example, the data identified by using the barometric pressure sensor 223 may be used to identify the altitude of the electronic device 200. The processor 210 may identify whether the altitude of the electronic device 200 is changed based on the data identified by using the barometric pressure sensor 223.

In operation 540, the processor 210 may identify whether the air pressure change amount (i.e., the amount the air pressure has changed) is less than a first air pressure change amount. The processor 210 may identify whether the air pressure change amount during the designated time interval (e.g. a time period) is less than the first air pressure change amount. For example, the processor 210 may identify whether the air pressure change amount at the end of the designated time interval is less than the first air pressure change amount. The processor 210 may identify that the air pressure decreases during the designated time interval, based on identifying that the air pressure change amount during the designated time interval is less than the first air pressure change amount.

In operation 550, in case that the air pressure change amount is less than the first air pressure change amount, the processor 210 may identify that the user is walking uphill. The processor 210 may identify that the user is walking uphill based on identifying that the air pressure change amount is less than the first air pressure change amount. In some examples, the air pressure change amount being a negative value (e.g., indicating a decrease in the air pressure between the start of the designated time interval and the end of the designated time interval) may be interpreted to indicate that the user is walking uphill. In other examples, for it to be interpreted that the user is walking uphill, the air pressure change amount is required to be less than the first air pressure change amount (which may itself be a negative value), thereby requiring the air pressure change amount to have at least a specified magnitude as opposed to simply being below zero. According to above example, the processor 210 may filter out cases where the user is walking on substantially flat land with merely mild undulation that result in a slight decrease in air pressure over the designated time interval, as opposed to when the user is walking a more-generally uphill path.

In operation 560, in case that the air pressure change amount is equal to or greater than the first air pressure change amount, the processor 210 may identify whether the air pressure change amount is equal to or greater than a second air pressure change amount. For example, the processor 210 may identify whether the air pressure change amount is equal to or greater than the second air pressure change based on identifying that the air pressure change amount is equal to or greater than the first air pressure change amount. For example, the processor 210 may identify whether the air pressure change amount during the designated time interval is equal to or greater than the second air pressure change amount. For example, the processor 210 may identify whether the air pressure change amount at the end of the designated time interval is more than the second air pressure change amount. The processor 210 may identify that the air pressure increases during the designated time interval based on identifying that the air pressure change amount during the designated time interval is equal to or greater than the second air pressure change amount.

In operation 570, in case that the air pressure change amount is equal to or greater than the second air pressure change amount, the processor 210 may identify that the user is walking downhill. The processor 210 may identify that the user is walking downhill based on identifying that the air pressure change amount is equal to or greater than the second air pressure change amount. In some examples, the air pressure change amount being a positive value (e.g., indicating an increase in the air pressure between the start of the designated time interval and the end of the designated time interval) may be interpreted to indicate that the user is walking downhill. In other examples, for it to be interpreted that the user is walking downhill, the air pressure change amount is required to be more than the second air pressure change amount, thereby requiring the air pressure change amount to have at least a specified magnitude as opposed to simply being above zero. According to above example, the processor 210 may filter out cases where the user is walking on substantially flat land with merely mild undulation that result in a slight increase in air pressure over the designated time interval, as opposed to when the user is walking a more-generally downhill path.

In operation 580, in case that the air pressure change amount is less than the second air pressure change amount, the processor 210 may identify that the user is walking on a flatland. For example, the processor 210 may identify that the user is walking on the flatland based on identifying that the air pressure change amount is less than the second air pressure change amount. For example, the processor 210 may identify that the user is walking on the flatland based on identifying that the air pressure change amount is equal to or greater than the first air pressure change amount and less than the second air pressure change amount during the designated time interval. For instance, the air pressure change amount between the start and end of the designated time interval being equal to or greater than the first air pressure change amount and less than the second air pressure change amount may correspond to the case of the user walking on substantially flat land with only a mild undulation, if any.

FIGS. 6A to 6C illustrate examples of trends for identifying a walking environment of the user, according to an example of the disclosure.

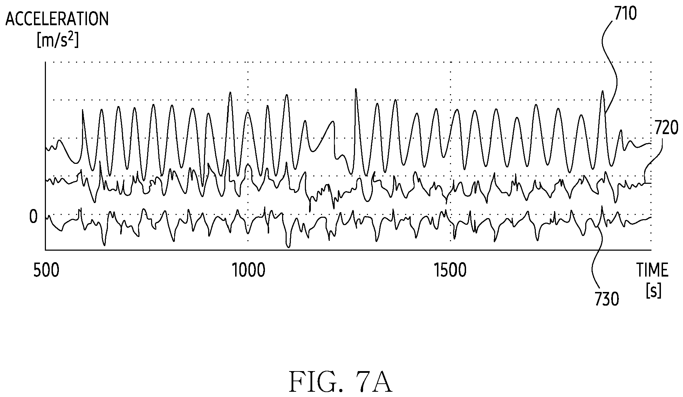

FIGS. 7A to 7C illustrate examples of trends for identifying a walking environment of the user, according to an example of the disclosure.

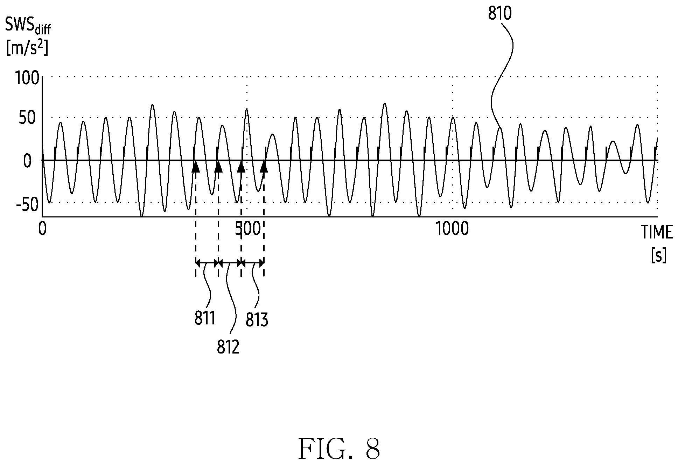

FIG. 8 illustrates an example of a trend for identifying a walking environment of the user, according to an example of the disclosure.

Referring to FIGS. 6A to 6C, trends 610 to 630 indicate changes in acceleration of the electronic device 200 over time. A processor 210 may identify the acceleration of the electronic device 200 for the 3 axes by using an acceleration sensor 221. The processor 210 may identify the trend 610 by monitoring the acceleration in the first direction (or the x-axis direction) over time. The processor 210 may identify the trend 620 by monitoring the acceleration in the second direction (or the y-axis direction) over time. The processor 210 may identify the trend 630 by monitoring the acceleration in the third direction (or z-axis direction) over time. The processor 210 may identify the magnitude of the acceleration in the 3 axis direction. The processor 210 may identify a trend indicating the magnitude of the acceleration in the 3 axis direction. For example, the processor 210 may identify the magnitude of the acceleration in the 3 axis direction by using Equation 1.

【Equation 1】

$$a_{NORM} = \sqrt{x^2 + y^2 + z^2}$$

Referring to the Equation 1, $a_{NORM}$ ($a_{NORM}$) is the magnitude of the acceleration in the 3 axis direction. x is the magnitude of the acceleration in the first direction. y is the magnitude of the acceleration in the second direction. The processor 210 may identify a trend indicating the magnitude of the acceleration over time based on identifying the magnitude of the acceleration in the 3 axis direction at a specific timing.

According to an example, the processor 210 may identify the difference of the sliding window summing based on trends of the acceleration in the 3 axis direction over time. The processor 210 may identify a trend 640 indicating a change in the difference of the sliding window summing over time. For example, the processor 210 may identify the sliding window summing by using Equation 2. The processor 210 may identify the difference of the sliding window summing by using the Equation 3.

【Equation 2】

$$SWS(k) = \sum_{i=k-N+1}^{k} a_{NORM}(i)$$

Referring to the Equation 2, SWS(k) indicates the sliding window summing for the magnitude of the acceleration in the 3 axis direction. The processor 210 may identify the SWS(k) by identifying the sliding window summing for the magnitude of the acceleration from the timing k-N+1 to the timing k.

【Equation 3】

$$SWS_{diff(k)} = SWS(k - N + 1) - SWS(k)$$

Referring to the Equation 3, $SWS_{diff}(k)$ indicates the difference of the sliding window summing for the magnitude of the acceleration in the 3 axis direction. The processor 210 may identify (or obtain) a difference between the sliding window summing of the timing k-N+1 and the sliding window summing of the timing k as a difference of the sliding window summing at the timing k.

According to an example, the processor 210 may identify the trend 640 by setting k and N values so that the trend of the difference of the sliding window summing is configured with a sine waveform. The processor 210 may identify at least one zero-crossing point based on the trend 640. A timing 641, a timing 642, and a timing 643 may be examples of zero-crossing points.

For example, the processor 210 may identify one step based on the zero-crossing point. The processor 210 may identify a gap between a first zero-crossing point (e.g., the timing 643) and a second zero-crossing point (e.g., the timing 642) identified before the first zero-crossing point, as a time interval indicating the one step. The processor 210 may identify the number of the zero-crossing points as the number of steps, in the trend 640. The processor 210 may identify that the user is walking, or moving by another form of step-based movement, based on identifying that the number of the zero-crossing points within a designated time interval is equal to or greater than a designated number.

According to an example, the processor 210 may identify an air pressure change amount (e.g., an air pressure difference) based on identifying that the user is walking. The processor 210 may identify a trend 650 of the air pressure over time within the designated time interval. The processor 210 may identify the air pressure change amount within the designated time interval, based on the trend 650. The processor 210 may identify that the air pressure change amount is equal to or greater than a first air pressure change amount and less than a second air pressure change amount within the designated time interval, based on the trend 650. The processor 210 may identify that the user is walking on the flatland based on identifying that the air pressure change amount is equal to or greater than the first air pressure change amount and less than the second air pressure change amount, within the designated time interval. For example, the processor 210 may identify that the user is walking on the flatland based on identifying that the air pressure is maintained (or substantially maintained, such as being maintained within a set range) within the designated time interval.

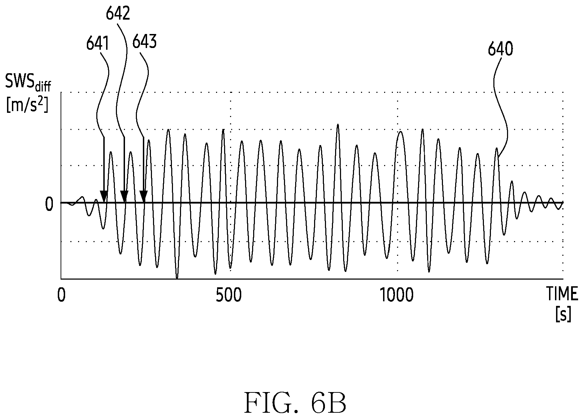

Referring to FIGS. 7A to 7C, trends 710 to 730 indicate changes in acceleration of the electronic device 200 over time. The trend 710 may correspond to the trend 610 of FIG. 6A. The trend 720 may correspond to the trend 620 of FIG. 6A. The trend 730 may correspond to the trend 630 of FIG. 6A. By identifying the magnitude of the acceleration in the 3 axis direction based on the trends 710 to 730, the processor 210 may identify a trend 740 indicating the change in the difference of the sliding window summing over time. The trend 740 may correspond to the trend 640 of FIG. 6B.

According to an example, the processor 210 may identify at least one zero-crossing point based on the trend 740. The processor 210 may identify the number of the zero-crossing points as the number of the steps, in the trend 740. The processor 210 may identify that the user is walking based on identifying that the number of the zero-crossing points within a designated time interval is equal to or greater than the designated number.

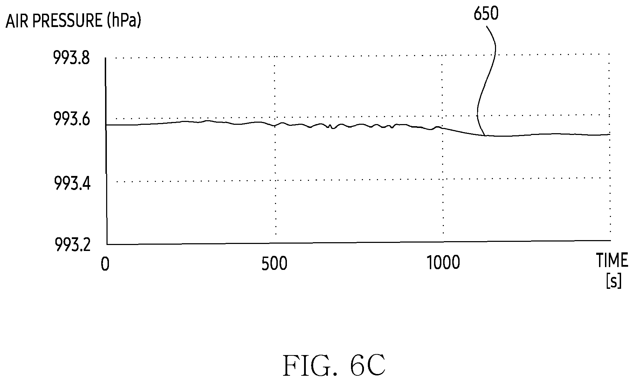

According to an example, the processor 210 may identify the air pressure change amount based on identifying that the user is walking. The processor 210 may identify a trend 750 of the air pressure over time within the designated time interval. The trend 750 may correspond to the trend 650 of FIG. 6C, e.g. in the sense of measuring air pressure change over time. The processor 210 may identify that the air pressure change amount is equal to or greater than the second air pressure change amount within the designated time interval, based on the trend 750. The processor 210 may identify that the user is walking downhill based on identifying that the air pressure change amount is equal to or greater than the second air pressure change amount within the designated time interval. For example, the processor 210 may identify that the user is walking downhill based on identifying that the air pressure increases within the designated time interval.

Although not illustrated, the processor 210 may identify that the air pressure change amount is less than the first air pressure change amount within the designated time interval. The processor 210 may identify that the user is walking uphill based on identifying that the air pressure change amount is less than the first air pressure change amount within the designated time interval. For example, the processor 210 may identify that the user is walking uphill based on identifying that the air pressure decreases within the designated time interval.

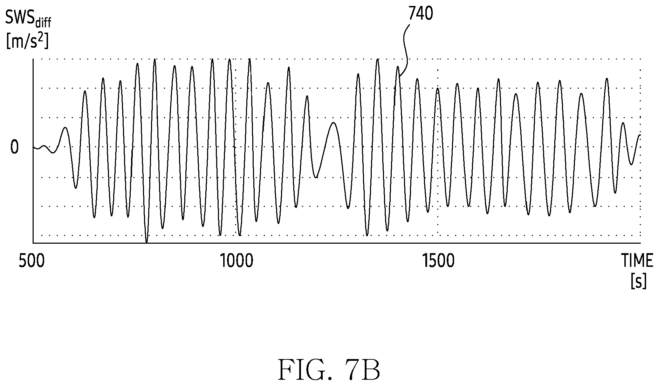

Referring to FIG. 8, by identifying the magnitude of the acceleration in the 3 axis direction, the processor 210 may identify a trend 810 indicating the change in the difference of the sliding window summing over time. The trend 810 may correspond to the trend 640 of FIG. 6B or the trend 740 of FIG. 7B.

According to an example, the processor 210 may identify at least one zero-crossing point based on the trend 810. The processor 210 may identify a time interval between the zero-crossing points. For example, the processor 210 may identify a time interval 811, a time interval 812, and a time interval 813. The processor 210 may identify a plurality of time intervals including the time interval 811, the time interval 812, and the time interval 813.

According to an example, the processor 210 may identify a walking frequency based on the plurality of time intervals. The processor 210 may identify the walking frequency based on a representative value (e.g., an average value or an intermediate value) of the plurality of time intervals. For example, the processor 210 may identify the representative value of the plurality of time intervals as the time interval 811. The processor 210 may identify the walking frequency based on the time interval 811. For example, the processor 210 may identify the walking frequency by using Equation 4.

【 Equation 4】

$$f = \frac{1}{te}$$

Referring to the Equation 4, f is the walking frequency. te is the representative value of the plurality of time intervals. The processor 210 may identify the walking speed of the user by identifying the walking frequency by using the representative value of the plurality of time intervals.

FIG. 9 illustrates an example of an operation of an electronic device according to an example.

Referring to FIG. 9, in operation 910, a processor 210 may obtain second data. For example, the processor 210 may obtain the second data by using at least one of a sensor 220 and/or a communication circuit 240. For example, the processor 210 may obtain the second data by using at least one of an acceleration sensor 221, a gyroscope sensor 222, and a barometric pressure sensor 223, and the communication circuit 240. For example, the processor 210 may obtain the second data and/or third data based on pre-processing data obtained through the sensor 220. For example, the second data and the third data may include at least one of a posture change for hand motion, a frequency characteristic, and a size (e.g., magnitude, extent etc.) and/or direction of movement for the hand motion. For example, the hand motion may mean a state in which the user holds (or grips) the electronic device 200. The processor 210 may identify at least one of the posture change for the hand motion (e.g., a change in how the user holds the electronic device), the frequency characteristic, and a size and/or direction of the movement for the hand motion by identifying the movement of the user while the user holds the electronic device 200.

For example, the second data and the third data may include at least one of data related to acceleration of the electronic device 200 obtained through the acceleration sensor 221, data related to angular velocity of the electronic device 200 obtained through the gyroscope sensor 222, data related to air pressure around the electronic device 200 obtained through the barometric pressure sensor 223, and information on weather obtained from an external electronic device through the communication circuit 240.

The second data may mean data obtained at a second timing. The third data may mean data obtained at a first timing earlier than the second timing.

In operations 920 and 930, the processor 210 may learn the second data and/or the third data. The processor 210 may set a plurality of parameters of a designated model 940 based on learning the second data and/or the third data. For example, the designated model 940 may include an artificial intelligence model. For example, the designated model 940 may be configured (or designed) based on machine learning such as decision trees (DTs), support vector machine (SVM), k-nearest neighbor (KNN), and/or multi-layer perceptron (MLP) and the like.

For example, the third data may be obtained according to a walking history of the user. The processor 210 may obtain the third data based on the walking history of the user. The first timing at which the third data is obtained may be different from the second timing at which the second data is obtained. The first timing at which the third data is obtained may be earlier than the second timing at which the second data is obtained. For example, the processor 210 may set (or change or update) the designated model 940 by learning the third data obtained at the first timing. The processor 210 may set (or change or update) the plurality of parameters of the designated model 940 by using the third data obtained at the first timing. The processor 210 may set the designated model 940 by learning the second data obtained at the second timing. The processor 210 may set (or change or update) the plurality of parameters of the designated model 940 by using the second data obtained at the second timing.

According to an example, the processor 210 may use at least a part or all of the second data for learning the designated model 940. The processor 210 may set at least a part or all of the second data as input data of the designated model 940. For example, the processor 210 may learn the second data while the operation mode of the designated model 940 is in the learning mode (or training phase). For example, the processor 210 may set the second data as the input data of the designated model 940 while the operation mode of the designated model 940 is in the identification mode (or recognition phase).

In operation 950, the processor 210 may obtain information on a moving state of the electronic device 200. For example, the processor 210 may obtain information on the moving state of the electronic device 200 based on setting at least a part or all of the second data as the input data of the designated model 940. For example, the information on the moving state of the electronic device 200 may include information on the posture in which the user carries the electronic device 200. Accordingly, in various examples a method may follow operations 910, 940 and 950, where the obtained second data is used as input data of the designated model 940 for the obtaining of information on the moving state; while in other examples a method may follow operations 910, 920, 930 and 940, where the obtained second data and/or third data is learned, parameters of the designated model are set using the second data and/or the third data, and the designated model 940 may be set based on the parameters (further, operation 950 may also be performed with the second data also used as input data for the designated model 940).

For example, the processor 210 may identify that the user carries the electronic device 200 by using a hand of the user based on setting at least a part or all of the second data as the input data of the designated model 940.

For example, the processor 210 may identify that the user carries the electronic device 200 by using a pocket based on setting at least a part or all of the second data as the input data of the designated model 940.

For example, the processor 210 may identify that the user is swing his arm in a state of holding electronic device 200, based on setting at least a part or all of the second data as the input data of the designated model 940.

FIG. 10 is a flowchart illustrating an operation of an electronic device, according to an example.

Referring to FIG. 10, in operation 1010, the processor 210 may identify (or detect, determine, obtain etc.) first steps (e.g., a first plurality of steps) of the user. For example, the processor 210 may identify the first steps of the user based on the magnitude of acceleration in the 3 axis direction by using an acceleration sensor 221. For example, the processor 210 may identify a trend (e.g., a trend 640 of FIG. 6B and a trend 740 of FIG. 7B) indicating a change in the difference of the sliding window summing over time, based on the magnitude of the acceleration in the 3 axis direction. The processor 210 may identify the first steps of the user based on at least one zero-crossing point related to the identified trend.

According to an example, the processor 210 may identify the first steps by excluding steps identified as outliers from among the steps of the user. For example, the processor 210 may identify the first steps by excluding outliers among the steps of the user based on a z-score.

In operation 1020, the processor 210 may identify (or determine, detect, calculate etc.) whether the number of the first steps is equal to or greater than a designated number of times. For example, the processor 210 may identify whether the number of the first steps is equal to or greater than the designated number of times (e.g., 12 times) in order to identify a valid walking interval. The processor 210 may identify whether the steps of the user occur continuously equal to or greater than the designated number of times based on a trend indicating the change in the difference of the sliding window summing over time, identified based on the magnitude of the acceleration in the 3 axis direction. According to an example, the processor 210 may identify the number of the first steps as at least one valid walking interval according to the designated number of times.

According to an example, the processor 210 may perform (e.g., repeat) operation 1010 based on identifying (in operation 1020) that the number of the first steps is not equal to or greater than the designated number of steps. For example, the processor 210 may identify the first steps of the user again based on identifying that the number of the first steps (identified in a previous performance of operation 1010) is not equal to or greater than the designated number of times.

In operation 1030, the processor 210 may identify (or obtain, determine, detect etc.) feature values according to the first steps. For example, the processor 210 may identify the feature values according to the first steps based on identifying that the number of the first steps is equal to or greater than the designated number of times.

According to an example, the feature values may include values for indicating a walking pattern or walking stability. For example, the feature values may include values for the magnitude of the acceleration in the 3 axis direction. For example, the feature values may include at least one of a minimum value, a maximum value, an average value, a standard deviation, a root mean squared (RMS) value, a peak-to-peak (P2P) value, a Peak count value, a signal magnitude area (SMA) value, a mean amplitude deviation (MAD) value, and a coefficient of variance (COV) value.

At operation 1040, the processor 210 may identify (or obtain, detect, determine etc.) a population. For example, the processor 210 may identify the population based on the feature values according to the first steps. According to an example, the population may be configured with values related to a walking pattern according to a plurality of steps of the user. For example, the processor 210 may identify feature values according to the first steps and feature values according to second steps (e.g., a second plurality of steps) which occurred before the first steps were identified (e.g., prior to identifying the first steps in operation 1010, the second steps were identified, such as through an earlier performance of the method of FIG. 10). The processor 210 may identify values related to the walking pattern according to the first steps based on the feature values according to the first steps. The processor 210 may identify values related to the walking pattern according to the second steps based on the feature values according to the second steps. The processor 210 may configure the values related to the walking pattern according to the first steps and the values related to the walking pattern according to the second steps as the population including the values related to the walking pattern according to the plurality of steps (e.g., the population comprises both sets of values/feature values). The processor 210 may perform learning related to the walking pattern of the user based on the population. For example, the processor 210 may perform feature scaling by normalizing the population. The processor 210 may perform the learning related to the walking pattern of the user based on the feature-scaled data.

Hereinafter, a specific example of an operation of the processor 210 according to operations 1010 to 1040 will be described later.

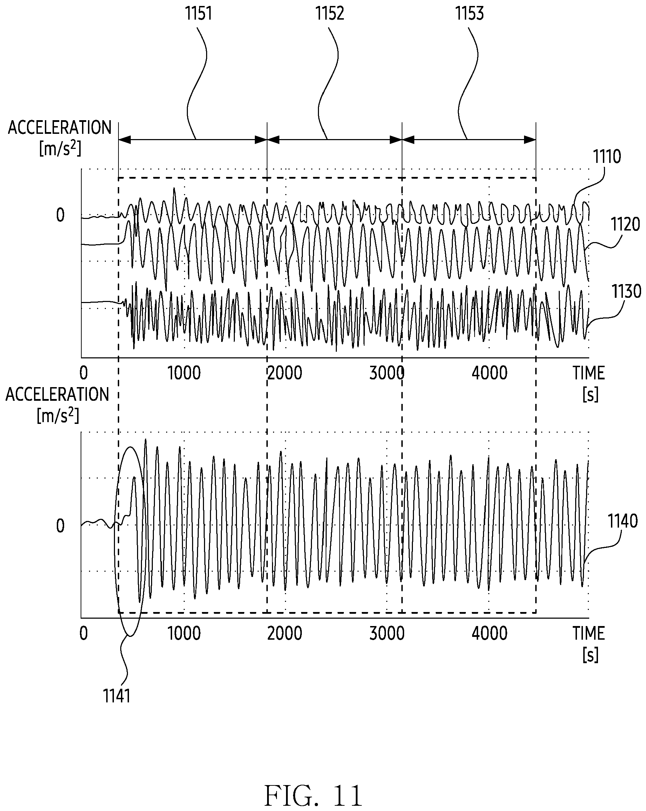

FIG. 11 illustrates an example of an operation of an electronic device according to an example.

FIG. 12 illustrates an example of an operation of an electronic device according to an example.

Referring to FIG. 11, a processor 210 may identify trends indicating acceleration in the 3 axis direction by using an acceleration sensor 221. For example, the processor 210 may identify a trend 1110 indicating acceleration in the first direction (or the x-axis direction). The processor 210 may identify a trend 1120 indicating acceleration in the second direction (or the y-axis direction). The processor 210 may identify a trend 1130 indicating acceleration in the third direction (or the z-axis direction). The processor 210 may identify a trend 1140 indicating a change in difference of sliding window summing based on the trends 1110 to 1130.

According to an example, the processor 210 may identify at least one zero-crossing point based on the trend 1140. The processor 210 may identify steps of a user based on identifying at least one zero-crossing point. The processor 210 may identify a valid walking interval based on the steps of the user.

According to an example, the processor 210 may identify the valid walking interval for each designated number of times (e.g., 10 times). For example, the processor 210 may identify a valid walking interval 1151, a valid walking interval 1152, and a valid walking interval 1153. The processor 210 may identify the valid walking interval for each designated number of times based on identifying that the number of the steps of the user is equal to or greater than the designated number of times.

According to an example, the processor 210 may identify first steps among steps configuring the valid walking interval 1151, except for a step 1141 identified as an outlier. The processor 210 may identify feature values according to the first steps. The processor 210 may identify the feature values of the steps configuring the valid walking interval 1152 and the valid walking interval 1153.

Referring to FIG. 12, the processor 210 may identify 10 feature values per step. The processor 210 may configure 10 feature values according to the first steps configured with 10 steps as a matrix 1210. The matrix 1210 may be configured with 10 by 10 (10 x 10 - i.e., 10 rows and 10 columns). The feature values configuring the matrix 1210 are exemplary and may be changed.

According to an example, the processor 210 may identify the feature values based on the trend 1140. The processor 210 may identify the feature values by using the difference of the sliding window summing configuring one step. For example, the processor 210 may classify a trend indicating the change in the difference of the sliding window summing configuring the one step into discrete values (e.g., n) according to a unit time interval. The processor 210 may identify feature values using the classified values. There now follows a number of examples of feature values, where it will be appreciated that any one of more of these types of features values may be implemented in the examples disclosed herein.

The values shown in FIG. 12 are exemplary values and may not be described according to examples described below.

For example, the first column may mean a minimum value (min). The processor 210 may identify the minimum value of the difference of the sliding window summing configuring the one step as the value of the first column.

For example, the second column may mean a maximum value (max). The processor 210 may identify the maximum value of the difference of the sliding window summing configuring the one step as the value of the second column.

For example, the third column may mean an average value (mean). The processor 210 may identify the average value of the difference of the sliding window summing configuring the one step as the value of the third column.

For example, the fourth column may mean a standard deviation. The processor 210 may identify the standard deviation of the difference of the sliding window summing configuring the one step as the value of the fourth column.

For example, the fifth column may mean a root mean squared (RMS) value. The processor 210 may identify the RMS value of the difference of the sliding window summing configuring the one step as the value of the fifth column.

For example, the sixth column may mean a peak-to-peak (P2P) value. The processor 210 may identify a difference between peak values of the trend indicating the difference of the sliding window summing configuring the one step as the value of the sixth column.

For example, the seventh column may mean a peak count value. The processor 210 may identify the number of peak values of the trend indicating the difference of the sliding window summing configuring the one step as the value of the seventh column.

For example, the eighth column may mean a signal magnitude area (SMA) value. The processor 210 may identify the SMA value of the trend indicating the difference of the sliding window summing configuring the one step as the value of the eighth column.

For example, the ninth column may mean a mean amplitude deviation (MAD) value. The processor 210 may identify the MAD value of the trend indicating the difference of the sliding window summing configuring the one step as the value of the ninth column.

For example, the tenth column may mean a coefficient of variance (COV) value. The processor 210 may identify the coefficient of variance (COV) value of the difference of the sliding window summing configuring the one step as the value of the tenth column.

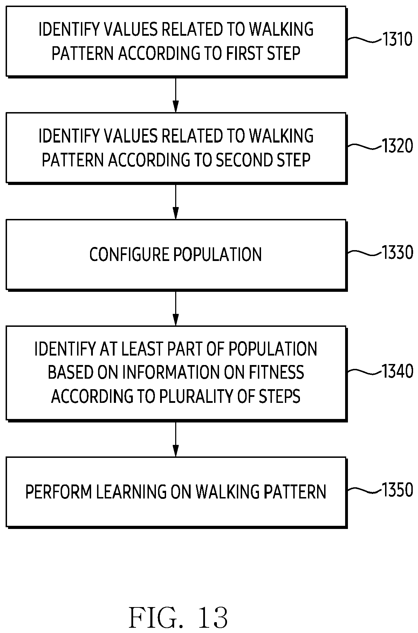

FIG. 13 is a flowchart illustrating an operation of an electronic device, according to an example.

Referring to FIG. 13, in operation 1310, the processor 210 may identify values related to walking patterns according to first steps. For example, the processor 210 may identify values related to the walking pattern according to the first steps based on the feature values according to the first steps. For example, the processor 210 may identify a first matrix configured with values related to the walking pattern according to the first steps by multiplying a matrix configured based on the feature values according to the first steps by a designated matrix.

In operation 1320, the processor 210 may identify values related to the walking pattern according to the second steps. For example, the processor 210 may identify values related to the walking pattern according to the second steps based on the feature values according to the second steps. For example, the processor 210 may identify a second matrix configured with values related to the walking pattern according to the second steps by multiplying a matrix configured based on the feature values according to the second steps by the designated matrix. For example, the values related to the walking pattern according to the second steps may be values learned according to the walking history of the user.

According to an example, as the feature values of the first steps and the feature values of the second steps increase, the amount of computation increases and the learning effect of the designated model may decrease. Accordingly, the processor 210 may reduce the dimension of the walking pattern by identifying various feature values as feature values according to the walking pattern.

In operation 1330, the processor 210 may configure a population. For example, processor 210 may configure values related to the walking pattern according to the first steps and values related to the walking pattern according to the second steps as the population including values related to the walking pattern according to a plurality of steps.

For example, the processor 210 may identify the values related to the walking pattern according to the first steps and the values related to the walking pattern according to the second steps as values related to the walking pattern according to the plurality of steps. The processor 210 may express the population including values related to the walking pattern according to the plurality of steps as a third matrix.

In operation 1340, the processor 210 may identify at least a part of the population based on information on fitness according to the plurality of steps. For example, the processor 210 may identify at least a part of the population based on information on the fitness according to the plurality of steps obtained based on the population.

According to an example, the processor 210 may obtain information on fitness according to the plurality of steps based on the population. For example, the processor 210 may identify a representative value of each column among the population expressed as the third matrix. For example, the processor 210 may identify the mean value of each column among the population expressed as the third matrix as the representative value. The processor 210 may obtain information on the fitness according to the plurality of steps based on identifying the mean value of each column among the population expressed as the third matrix as the representative value. The information on the fitness according to the plurality of steps may be expressed as a fourth matrix. For instance, in view of being formed from the mean value of each column, the information on the fitness may be considered to provide a general indication of the fitness level of the subject/user according to the recorded/determined/identified feature values, and/or may allow for identified of high fitness and/or low fitness for the subject/user (e.g., occasions on which walking is more or less intensive than average).

According to an example, the processor 210 may identify at least a part of the population based on the information on the fitness according to the plurality of steps. The processor 210 may identify at least a part of the population in order of high fitness according to the plurality of steps. For example, high fitness may refer to features values above the mean values (i.e., above the information on the fitness).

In operation 1350, the processor 210 may perform learning on the walking pattern. For example, the processor 210 may perform the learning on the walking pattern of the user based on at least a part of the population.

According to an example, the processor 210 may update the walking pattern by repeating the learning on the walking pattern. The processor 210 may identify that the walking pattern has been sufficiently learned based on identifying that the change amount of the walking pattern according to learning on the walking pattern is within the reference range. The processor 210 may stop learning on the walking pattern based on identifying that the walking pattern has been sufficiently learned.

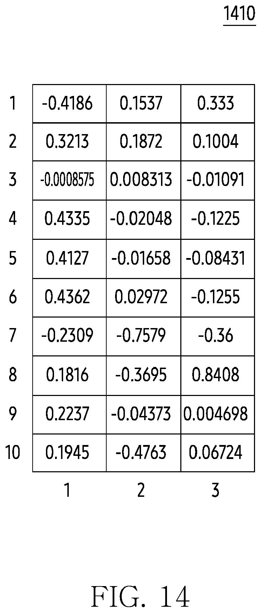

A specific example of operations according to operations 1310 to 1350 will be described later with reference to FIGS. 14 to 18.

FIG. 14 illustrates an example of a designated matrix according to an example.

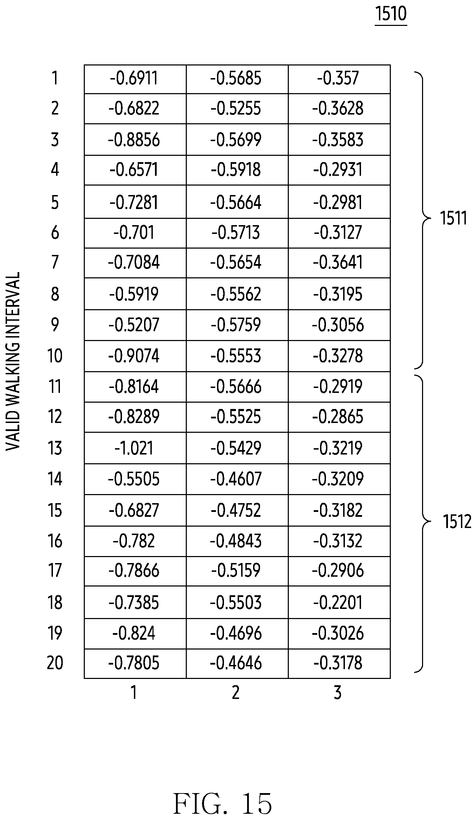

FIG. 15 illustrates an example of a matrix indicating a population according to an example.

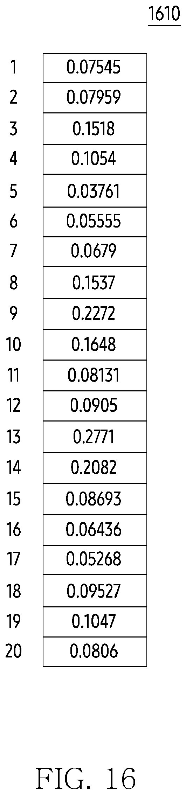

FIG. 16 illustrates an example of a matrix indicating information on fitness according to an example.

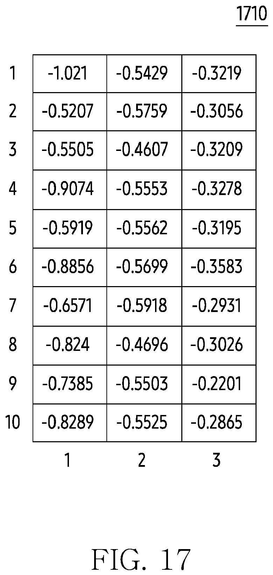

FIG. 17 illustrates an example of a matrix indicating at least a part of a population according to an example.

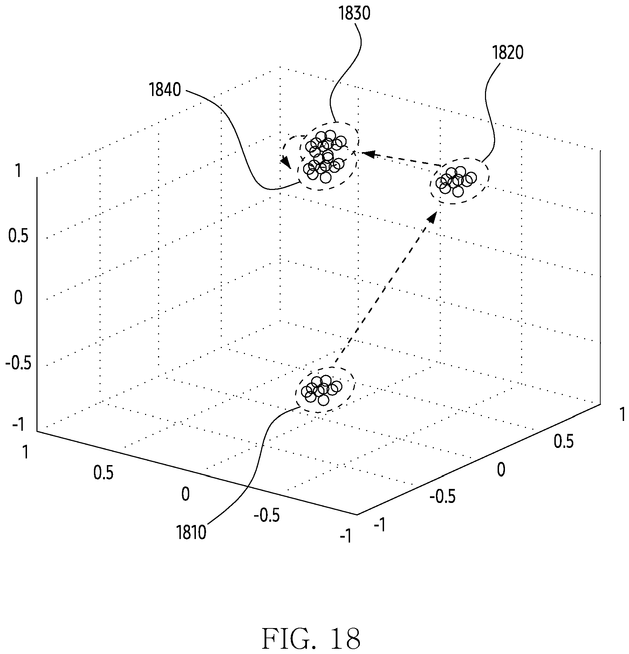

FIG. 18 illustrates an example of a learning result for a walking pattern according to an example.

Referring to FIG. 14, a processor 210 may identify a matrix 1410. For example, the matrix 1410 may be stored in a memory 230. For example, the matrix 1410 may be in a pre-modeled state or an initial state. According to an example, the processor 210 may receive the matrix 1410 from an external electronic device (e.g., a server) connected to an electronic device 200.

According to an example, the matrix 1410 may be used to identify values related to walking pattern according to first steps. The matrix 1410 may be used to identify values related to walking patterns according to second steps.

Referring to FIG. 15, the processor 210 may identify the values related to the walking patterns according to the first steps by multiplying the matrix 1410 (e.g., the matrix 1210 of FIG. 12) indicating feature values for the first steps. For example, the processor 210 may identify the values related to the walking patterns according to the first steps by multiplying (e.g., by matrix multiplication) the matrix 1410 with a matrix indicating feature values for the first steps (such as matrix 1210). The processor 210 may indicate values related to the walking pattern according to the first steps as a matrix 1512(or a part of a larger matrix 1510). For example, the number of first steps may be 10 times. The matrix 1512 may be configured with 10 x 3 (i.e., 10 rows and 3 columns).

The processor 210 may identify the values related to the walking pattern according to the second steps by multiplying the matrix 1410 indicating the feature values for the second steps. For example, the processor 210 may identify the values related to the walking pattern according to the second steps by multiplying (e.g., by matrix multiplication) the matrix 1410 with a matrix indicating features values for the second steps. The processor 210 may indicate the values related to the walking pattern according to the second steps as a matrix 1511 (or a part of a larger matrix 1510). For example, the number of second steps may be 10 times. The matrix 1511 may be configured with 10 x 3. For example, the second steps may occur before the first steps are identified. For example, the second steps may be pre-learned steps before the first steps are identified.

According to an example, the processor 210 may configure the population. The processor 210 may configure the values related to the walking pattern according to the first steps and the values related to the walking pattern according to the second steps as the population including values related to the walking pattern according to the plurality of steps. For example, the processor 210 may identify the matrix 1510 by combining the matrix 1512, which includes the values related to the walking pattern according to the first steps, and the matrix 1511, which includes the values related to the walking pattern according to the second steps.

According to an example, the matrix 1510 may be configured with the values related to the walking pattern according to the plurality of steps. The row of the matrix 1510 may mean the plurality of steps. The plurality of steps may be 20 times. The matrix 1510 may be configured with 20 x 3 (i.e. 20 rows and 3 columns).

Referring to FIG. 16, the processor 210 may indicate each row in the matrix 1510 as a 3 dimensional coordinate system. The first column of the matrix 1510 may mean a coordinate value of the x-axis. The second column of the matrix 1510 may mean a coordinate value of the y-axis. The third column of the matrix 1510 may mean a coordinate value of the z-axis. For example, the first row of the matrix 1510 may correspond to a first coordinate. The second row of the matrix 1510 may correspond to a second coordinate etc.. The processor 210 may identify the first to twentieth coordinates based on the matrix 1510. The processor 210 may identify an average coordinate of the first to twentieth coordinates. The processor 210 may identify a Euclidean distance between each of the first to twentieth coordinates and the average coordinate. The processor 210 may identify information on fitness according to a plurality of steps by identifying the Euclidean distance between each coordinate from the first coordinate to the twentieth coordinate and the average coordinate. The processor 210 may indicate the information on fitness according to the plurality of steps as a matrix 1610. For example, each row of the matrix 1610 may mean the fitness for each step. For example, the first row of the matrix 1610 may mean the fitness for the first step of the second steps. For example, the fitness may be used to identify values of a matrix, representing a user's gait pattern well.

Referring to FIG. 17, the processor 210 may identify at least a part of the population based on information on the fitness according to the plurality of steps. For example, the processor 210 may identify at least a part of the population by identifying top 10 steps having the high fitness. The processor 210 may indicate, values related to the walking pattern according to the top 10 steps having(or exhibiting) the high fitness, as a matrix 1710.

Referring to FIG. 18, the processor 210 may perform learning on the walking pattern of the user by performing the operation according to FIGS. 14 to 17.

According to an example, the processor 210 may identify 10 first coordinates 1810 corresponding to the matrix 1710. The processor 210 may identify the second coordinates 1820 by performing learning on the walking pattern by using values related to the walking pattern included in the matrix 1710 and values related to the newly obtained walking pattern. The processor 210 may identify the Euclidean distance between the representative value of the first coordinates 1810 and the second coordinates 1820. The processor 210 may identify the Euclidean distance between the representative value of the first coordinates 1810 and the second coordinates 1820 as the change amount of the walking pattern according to learning. The processor 210 may identify whether the change amount of the walking pattern according to learning is within a reference range.

According to an example, the processor 210 may perform learning on the walking pattern whenever a valid walking interval is identified. The processor 210 may evolutionarily learn the walking pattern of the user. For example, the processor 210 may repeat learning on the walking pattern based on identifying that the change amount of the walking pattern according to learning is outside the reference range.

For example, the processor 210 may identify the third coordinates 1830 according to learning on the walking pattern. The processor 210 may identify the Euclidean distance between the representative value of the second coordinates 1820 and the representative value of the third coordinates 1830 as the change amount of the walking pattern according to learning. The processor 210 may identify that the change amount of the walking pattern according to learning is outside the reference range. The processor 210 may identify fourth coordinates according to learning on the walking pattern. The processor 210 may identify the Euclidean distance between the representative value of the third coordinates 1830 and the representative value of the fourth coordinates 1840 as the change amount of the walking pattern according to learning. The processor 210 may identify that the change amount of the walking pattern (relating to the third coordinates 1830 and the fourth coordinates 1840) according to learning is within the reference range. The processor 210 may identify that the walking pattern has been sufficiently learned based on identifying that the change amount of the walking pattern according to learning is within the reference range. The processor 210 may stop learning on the walking pattern based on identifying that the walking pattern has been sufficiently learned.

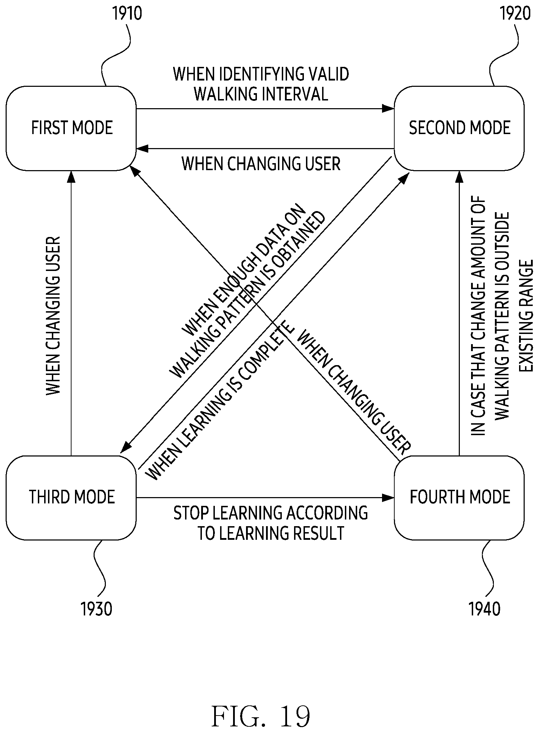

FIG. 19 illustrates an example of an operation mode of a processor according to an example.

Referring to FIG. 19, a processor 210 may operate in one of a first mode 1910, a second mode 1920, a third mode 1930 and a fourth mode 1940. For example, the processor 210 may learn a walking pattern based on the operation mode.

According to an example, the processor 210 may initialize fourth information on a walking history of a user and a second walking pattern stored in a memory 230 based on the first mode 1910. For example, the second walking pattern may be identified based on the walking history of the user. The second walking pattern may mean a learned walking pattern. For example, in the first mode 1910, the processor 210 may operate in a state in which the walking pattern is not learned. For example, the first mode 1910 may be referred to as an idle mode (or an idle state).

According to an example, the processor 210 may obtain data on the walking pattern of the user by using a sensor 220 based on the second mode 1920. For example, the processor 210 may obtain the data on the walking pattern of the user in order to learn the walking pattern in the second mode 1920. For example, in the second mode 1920, the processor 210 may identify a valid walking interval. The processor 210 may obtain the data on the walking pattern by identifying feature values related to steps in the valid walking interval.

According to an example, the processor 210 may learn the data on the walking pattern of the user based on the third mode 1930. For example, in the third mode 1930, the processor 210 may learn the data on the walking pattern obtained while operating in the second mode 1920. In case that the data on the walking pattern is sufficiently obtained for learning, the processor 210 may learn the data on the obtained walking pattern in the third mode 1930. For example, the processor 210 may update the walking pattern by learning the normal walking pattern of the user. The processor 210 may identify the learned walking pattern as a second walking pattern.

According to an example, the processor 210 may stop an operation of learning the data on the walking pattern of the user based on the fourth mode 1940. For example, the processor 210 may stop an operation of learning the data on the walking pattern of the user based on identifying that the change amount of the walking pattern according to learning is within the reference range.

According to an example, the processor 210 may set the operation mode of the processor 210 to the first mode 1910 based on identifying that a user of an electronic device 200 is changed. The processor 210 may initialize fourth information on the second walking pattern, which is the walking pattern learned in the first mode 1910. For example, the processor 210 may identify that the user is changed based on identifying that at least one of the gender and name of the user is changed in the first mode.

According to an example, the processor 210 may change the operation mode of the processor 210 from the first mode 1910 to the second mode 1920 in response to identifying the valid walking interval while operating in the first mode 1910.

According to an example, the processor 210 may change the operation mode of the processor 210 from the second mode 1920 to the third mode 1930 in response to identifying that enough data on the walking pattern has been obtained for learning while operating in the second mode 1920.

According to an example, the processor 210 may change the operation mode of the processor 210 from the third mode 1930 to the second mode 1920 in response to the completion of learning of the walking pattern while operating in the third mode 1930. According to an example, the processor 210 may change the operation mode of the processor 210 from the third mode 1930 to the second mode 1920 based on the learning result of the walking pattern while operating in the third mode 1930. For example, the processor 210 may change the operation mode of the processor 210 from the third mode 1930 to the second mode 1920 based on identifying that the change amount of the walking pattern is within the reference range. For example, the processor 210 may change the operation mode of the processor 210 from the third mode 1930 to the second mode 1920 based on identifying that the number of times the walking pattern has been learned (or evolved) is equal to or greater than the reference number. For example, the processor 210 may change the operation mode of the processor 210 from the third mode 1930 to the second mode 1920 based on identifying that the time at which the walking pattern is learned is equal to or greater than the reference time.

According to an example, the processor 210 may change the operation mode of the processor 210 from the fourth mode 1940 to the first mode 1910 based on identifying that the body profile of the user has been changed(e.g., that the user has changed), while operating in the fourth mode 1940. According to an example, the processor 210 may change the operation mode of the processor 210 from the fourth mode 1940 to the second mode 1920 based on identifying that the change amount of the walking pattern is outside the reference range while operating in the fourth mode 1940.

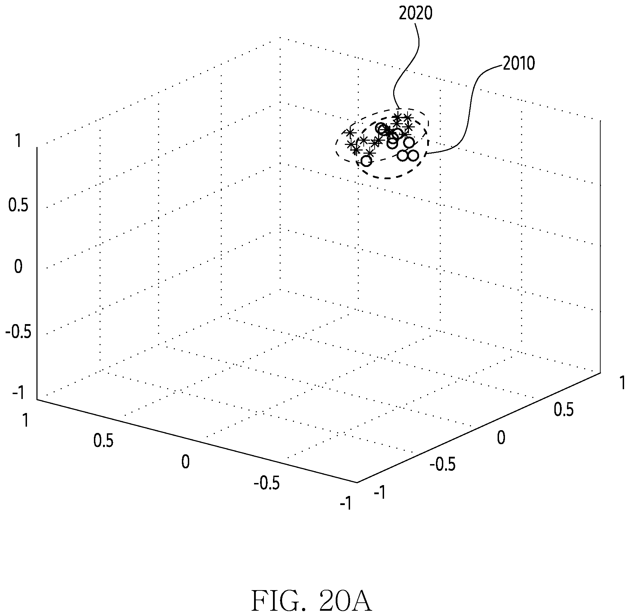

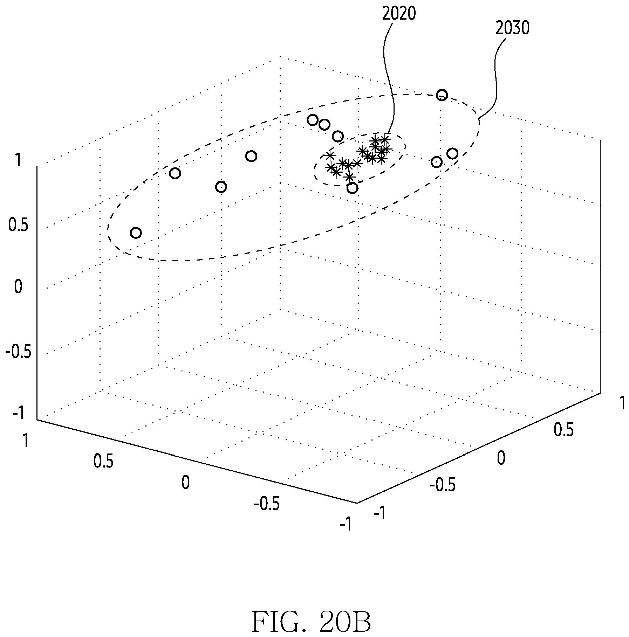

FIGS. 20A and 20B illustrate an example in which values for a walking pattern according to an example are displayed as three-dimensional coordinates.

According to an example, since each user has a different walking pattern (or walking habit), the processor 210 may not determine that the probability of the fall occurrence is high even in case that the walking pattern of the user is different from a reference walking pattern (e.g., the walking pattern of a general user). The processor 210 may change the probability of the fall occurrence according to the walking environment of the user.

According to an example, the processor 210 may identify the probability value that a fall occurs (i.e., the user has fallen or falls) based on a first value indicating the difference between the first walking pattern of the user and the reference walking pattern, a second value indicating the difference between the first walking pattern of the user and the second walking pattern of the user (e.g., the learned walking pattern), and second information on the walking environment of the user.

For example, the processor 210 may identify the first similarity based on the first value indicating the difference between the first walking pattern and the reference walking pattern. The processor 210 may identify the second similarity based on the second value indicating the difference between the first walking pattern and the second walking pattern. The processor 210 may identify the probability value that the fall occurs to the user based on the first similarity, the second similarity, and second information on the walking environment of the user.

For example, the processor 210 may identify the probability value that the fall occurs to the user by using Equation 5.

【 Equation 5】

$$S = \alpha \cdot S' + \beta \cdot S_3 + \gamma \cdot E, \quad \begin{cases} if\ S_1 \geq thrd, & S' = 0 \\ else, & S' = S_2 \end{cases}$$

Referring to Equation 5, " S " is the probability value that the fall occurs to the user. " S' " may be set based on Si. The " $S_1$ " is a similarity between the second walking pattern and the reference walking pattern. In case that the " Si " is equal to or greater than a threshold value (" thrd " of Equation 5), S' may be set to 0. In case that Si is less than the threshold value, the S' may be set to $S_2$. The " $S_2$ " is the first similarity between the first walking pattern and the reference walking pattern. "$S_3$ " is the second similarity between the first walking pattern and the second walking pattern. " E " is a value for the walking environment identified based on the second information, alpha (" $\alpha$ "), beta (" $\beta$ "), and gamma (" y ") are coefficients. For example, the processor 210 may identify a value E for the walking environment based on the second information. The processor 210 may identify the value E for the walking environment based on the weather, gradient, walking frequency (or walking speed), and/or the moving state of the electronic device 200. For example, the processor 210 may identify the value E for the walking environment based on a fuzzy set. For example, the fuzzy set may be configured as shown in Table 2.

[Table 2]

| Set | Element |
|---|---|
| W(weather) | w1(sunny), w2(cloudy), w3(rain), w4(snow) |
| S(gradient) | s1(flatland), s2(slope way) |
| F(walking frequency) | $f_1$(1.0),$f_2$(1.1), $f_3$(1.2), ...$f_n$(2.3) |
| P(moving state) | $p_1$(hand fixed), $p_2$(hand swing) |

Referring to Table 2, the processor 210 may configure one or more of the fuzzy set(s). The set W may include the element for the weather. The set S may include the element for the gradient. The set F may include the element for the walking frequency. The set P may include the element for the moving state. The processor 210 may identify at least a part of the element of the set W, the set S, the set F, and the set P based on fuzzy inference according to the fuzzy membership function. The processor 210 may identify the value E for the walking environment based on the identified element. Referring to FIG. 20A, the processor 210 may identify first coordinates 2010 according to values related to the first walking pattern, which is a current walking pattern. For example, the current walking pattern may be obtained in a normal walking state. The processor 210 may identify second coordinates 2020 according to values related to the second walking pattern, which is the reference walking pattern. The processor 210 may identify similarity based on the Euclidean distance between the representative value (e.g., average value) of the first coordinates 2010 and the representative value (e.g., average value) of the second coordinates 2020. For example, the shorter the Euclidean distance, the similarity may be identified as a value close to 1. The processor 210 may identify the similarity as a value close to 1 based on the Euclidean distance between the representative value of the first coordinates 2010 and the representative value of the second coordinates 2020.

Referring to FIG. 20B, the processor 210 may identify third coordinates 2030 according to values related to the first walking pattern, which is the current walking pattern. For example, the current walking pattern may be obtained in an abnormal walking state. For example, the current walking pattern may be obtained in a state that the user is injured or limping on one leg. The processor 210 may identify the second coordinates 2020 according to values related to the second walking pattern, which is the reference walking pattern. The processor 210 may identify the similarity based on the Euclidean distance between the representative value of the third coordinates 2030 and the representative value of the second coordinates 2020. For example, the processor 210 may identify the similarity as a value close to zero based on the Euclidean distance between the representative value (e.g., average value) of the third coordinates 2030 and the representative value (e.g., average value) of the second coordinates 2020.

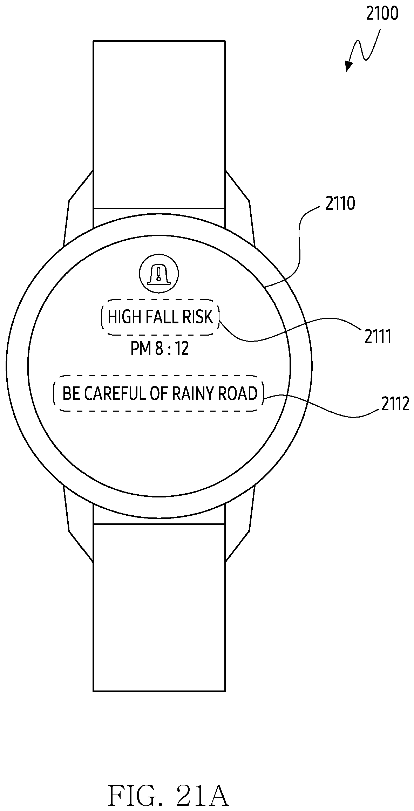

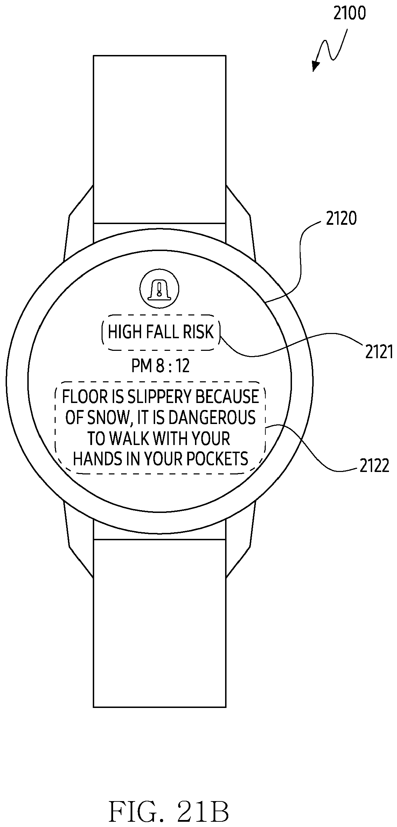

FIGS. 21A and 21B illustrate an example of a screen for warning of a fall hazard according to an example.

Referring to FIGS. 21A and 21B, a processor 210 may provide a notification for warning of a fall hazard in advance through an external electronic device 2100 connected to the electronic device 200. For example, the processor 210 may transmit a signal to the external electronic device 2100 to cause the external electronic device 2100 to provide the notification for warning of the fall hazard.

According to an example, the processor 210 may identify a probability value that a fall occurs or may occur for the user. The processor 210 may identify the threshold value as one of a plurality of candidate threshold values based on the identified probability value. For example, the processor 210 may identify the fall hazard degree as one of 'low', 'normal', 'high', and 'very high' based on the probability value that a fall occurs or may occur for the user. The processor 210 may identify one of the plurality of candidate threshold values according to the fall hazard degree. For example, the plurality of candidate threshold values according to the fall hazard degree may be set as shown in Table 1 described above.

According to an example, the processor 210 may provide the notification for warning the user of the fall hazard in advance based on that the probability value that a fall occurs or may occur for the user satisfies a designated condition. According to an example, the processor 210 may display the notification for warning the fall hazard to the user by using a display of the electronic device 200.

Referring to FIG. 21A, the processor 210 may display a screen 2110 by using the display of the external electronic device 2100. The screen 2110 may include a text 2111 indicating the fall hazard degree and a text 2112 indicating a warning related to a walking environment. For example, the text 2112 may be configured to represent information on the walking environment and information on a risk factor. For example, the text 2112 may be configured to indicate information on the walking environment and information on the risk factor. The text 2112 may be displayed to inform the risk on the rainy road. While FIG. 21A shows a wearable device as the external electronic device 2100, the external electronic device is not limited thereto and may take the form of other types of electronic device.

Referring to FIG. 21B, the processor 210 may display a screen 2120 by using the display of the external electronic device 2100. The screen 2120 may include a text 2121 indicating the fall hazard degree and a text 2122 indicating the warning related to the walking environment. For example, the text 2122 may be configured to indicate information on the walking environment and information on the risk factor. The text 2122 may be displayed to guide a behavior of the user on a snowy day.

According to an embodiment, the electronic device 200 may comprise at least one sensor 220, a memory 230, and a processor 210. The processor 210 may be configured to obtain, using the at least one sensor 220, first information related to a first walking pattern of a user of the electronic device 200 and second information related to a walking environment of the user. The processor 210 may be configured to identify, based on the first information and third information related to a reference walking pattern which is stored in the memory 230, a first value indicating a difference between the first walking pattern and the reference walking pattern. The processor 210 may be configured to identify, based on the first information and fourth information related to a second walking pattern of the user, which is stored in the memory 230 and identified based on a walking history of the user, a second value indicating a difference between the first walking pattern and the second walking pattern. The processor 210 may be configured to identify, based on the first value, the second value, and the second information, a threshold value related to fall detection. The processor 210 may be configured to execute, based on comparison between the threshold value and a value obtained through the at least one sensor 220, a function for the fall detection.

According to an embodiment, the at least one sensor 220 may include an acceleration sensor 221, a gyroscope sensor 222, and a barometric pressure sensor 223. The processor 210 may be further configured to obtain, based on first data obtained using the acceleration sensor 220 during a designated time interval, the first information related to the first walking pattern. The processor 210 may be further configured to obtain, based on second data obtained using at least one of the acceleration sensor 221, the gyroscope sensor 222, the barometric pressure sensor 223, and the communication circuit 240, the second information related to the walking environment of the user.

According to an embodiment, the processor 210 may be further configured to obtain, during the designated time interval, trends of acceleration corresponding to each of 3 axis directions. the processor 210 may be further configured to identify, based on difference of sliding window summing identified based on the trends of acceleration, the first walking pattern of the user.

According to an embodiment, the second information related to the walking environment of the user may include at least one of information on a gradient of a moving path of the electronic device 200, information on a moving state of the electronic device 200, and information on weather during the designated time interval.

According to an embodiment, the processor 210 may be further configured to obtain the information on the moving state of the electronic device 200 by setting at least of the second data as input data of designated model indicated by a plurality of parameters, stored in the memory 230. The plurality of parameters of the designated model may be configured based on learning of third data obtained according to the walking history of the user.

According to an embodiment, the electronic device 200 may further comprise a communication circuit 240. The processor 210 may be further configured to receive, using the communication circuit 240, the third information related to the reference walking pattern from a server connected to the electronic device 200. The processor 210 may be further configured to store the third information in the memory 230. The third information may be obtained based on a plurality of walking patterns related to a plurality of users stored in the server.

According to an embodiment, the processor 210 may be further configured to identify, based on a Euclidean distance between values related to the first walking pattern and values related to the reference walking pattern, the first value. The processor 210 may be further configured to identify, based on a Euclidean distance between the values related to the first walking pattern and values related to the second walking pattern, the second value.

According to an embodiment, the processor 210 may be further configured to perform learning of a walking pattern of the user based on the walking history of the user. The processor 210 may be further configured to, based on performing the learning of the walking pattern of the user, obtain the fourth information related to the second walking pattern. The processor 210 may be further configured to store the fourth information in the memory 230.

According to an embodiment, the processor 210 may be further configured to identify, based on the walking history of the user, a population including values related to a walking pattern according to a plurality of steps of the user. The processor 210 may be further configured to identify at least part of the population based on information on fitness which is obtained based on the population according to the plurality of steps. The processor 210 may be further configured to perform, based on the at least part of the population, learning of a walking pattern of the user.

According to an embodiment, the processor 210 may further configured to identify, using the at least one sensor 220, first steps of the user. The processor 210 may further configured to, based on identifying that the number of the first steps is equal to or greater than a designated number, identify feature values according to the first steps. The processor 210 may further configured to, based on the feature values according to the first steps, organize the population.

According to an embodiment, the processor 210 may be further configured to identify, based on the feature values according to the first steps, values related to a walking pattern according to the first steps. The processor 210 may be further configured to identify, based on feature values according to second steps of the user occurred before the first steps are identified, which are stored in the memory 230, values related to a walking pattern according to the second steps. The processor 210 may be further configured to organize the values related to a walking pattern according to the first steps and the values related to a walking pattern according to the second steps into the population including values related to the walking pattern according to the plurality of steps.

According to an embodiment, the processor 210 may be further configured to operate one of first mode to fourth mode. The processor 210 may be further configured to, based on the first mode, initialize the walking history of the user and the fourth information related to the second walking pattern which are stored in the memory 230. The processor 210 may be further configured to, based on the second mode, obtain, using the at least one sensor 220, data related to a walking pattern of the user. The processor 210 may be further configured to, based on the third mode, perform learning of data related to the walking pattern of the user. The processor 210 may be further configured to, based on the fourth mode, cease performing learning of data related to the walking pattern of the user.

According to an embodiment, the processor 210 may be further configured to, based on the first value, the second value, and the second information, identify a probability value that the user falls. The processor 210 may be further configured to identify, based on the probability value, one of a plurality of candidate threshold values as the threshold value.

According to an embodiment, the electronic device 200 may further comprise a communication circuit 240. The processor 210 may be further configured to, based on the probability value and the second information, transmit, a signal for causing the external electronic device 2100 to provide a notification to warn of a fall hazard through the external electronic device 2100 connected to the electronic device 200 and worn by the user, to the external electronic device 2100.

According to an embodiment, a method of an electronic device 200 may comprise obtaining, using at least one sensor 220 of the electronic device 200, first information related to a first walking pattern of a user of the electronic device 200 and second information related to a walking environment of the user. The method may comprise identifying, based on the first information and third information related to a reference walking pattern which is stored in a memory 230 a first value indicating a difference between the first walking pattern and the reference walking pattern. The method may comprise identifying, based on the first information and fourth information related to a second walking pattern of the user, which is stored in the memory 230 and identified based on a walking history of the user, a second value indicating difference between the first walking pattern and the second walking pattern. The method may comprise identifying, based on the first value, the second value, and the second information, a threshold value related to fall detection. The method may comprise executing, based on comparison between the threshold value and a value obtained through the at least one sensor 220, a function for the fall detection.

According to an embodiment, the method may further comprise obtaining, based on first data obtained using an acceleration sensor 221 of the electronic device 200 during a designated time interval, the first information related to the first walking pattern. The method may comprise obtain, based on second data obtained using at least one of the acceleration sensor 221, a gyroscope sensor 222 of the electronic device 200, a barometric pressure sensor 223 of the electronic device 200, and a communication circuit 240 of the electronic device 200, the second information related to the walking environment of the user.

According to an embodiment, the method may comprise obtaining, during the designated time interval, trends of acceleration corresponding to each of 3 axis directions. The method may comprise identifying, based on difference of sliding window summing identified based on the trends of acceleration, the first walking pattern of the user.

According to an embodiment, the second information related to the walking environment of the user may include at least one of information on a gradient of a moving path of the electronic device 200, information on a moving state of the electronic device 200, and information on weather during the designated time interval.

According to an embodiment, the method may comprise identifying, based on a Euclidean distance between values related to the first walking pattern and values related to the reference walking pattern, the first value. The method may comprise identifying, based on a Euclidean distance between the values related to the first walking pattern and values related to the second walking pattern, the second value.

According to an embodiment, the method may comprise performing learning of a walking pattern of the user based on the walking history of the user. The method may comprise based on performing the learning of the walking pattern of the user, obtaining the fourth information related to the second walking pattern. The method may comprise storing the fourth information in the memory 230.

According to an embodiment, the processor 210 may identify a fall hazard degree before a fall accident occurs to the user. The processor 210 may provide a notification for warning the fall hazard to the user based on the fall hazard degree. In case that the fall hazard degree is high, the processor 210 may change the sensitivity of the fall detection algorithm. For example, the processor 210 may adaptively change (or adjust) a threshold value related to fall detection. An electronic device according to various embodiments disclosed in the present document may be various types of devices.

It will be appreciated that the disclosure also includes examples, embodiments, aspects etc. in accordance with the following numbered paragraphs (numerals relating to the figures given in these paragraphs are for illustrative purposes and should not be viewed as limiting):

Paragraph 1. There is provided an electronic device 200 comprising at least one sensor 220, a memory 230, and a processor 210; wherein the processor 210 may be configured to obtain (or detect, identify etc.), using the at least one sensor 220, first information related to a first walking pattern of a user of the electronic device 200 and obtain (or detect, identify etc.) second information related to a walking environment of the user; wherein the processor 210 may be configured to identify, based on the first information and third information related to a reference walking pattern which is stored in the memory 230, a first value indicating a difference between the first walking pattern and the reference walking pattern; wherein the processor 210 may be configured to identify, based on the first information and fourth information related to a second walking pattern of the user, which is stored in the memory 230, a second value indicating a difference between the first walking pattern and the second walking pattern; wherein the processor 210 may be configured to identify, based on the first value, the second value, and the second information, a threshold value related to fall detection; and wherein the processor 210 may be configured to execute, based on comparison between the threshold value and a value obtained through the at least one sensor 220, a function for the fall detection.

Paragraph 2. The electronic device 200 of Paragraph 1, wherein the fourth information related to the second walking pattern of the user is identified based on a walking history of the user.

Paragraph 3. The electronic device 200 of Paragraph 2, wherein the electronic device 200 may include a communication circuit 240; wherein the at least one sensor 220 may include an acceleration sensor 221, a gyroscope sensor 222, and a barometric pressure sensor 223; wherein the processor 210 may be further configured to obtain, based on first data obtained using the acceleration sensor 220 during a designated time interval, the first information related to the first walking pattern; and/or wherein the processor 210 may be configured to obtain, based on second data obtained using at least one of the acceleration sensor 221, the gyroscope sensor 222, the barometric pressure sensor 223, and the communication circuit 240, the second information related to the walking environment of the user. Optionally, the processor 210 may be configured to receive, using the communication circuit 240, the third information related to the reference walking pattern from a server connected to the electronic device 200; wherein the processor 210 may be further configured to store the third information in the memory 230 and wherein the third information may be obtained based on a plurality of walking patterns related to a plurality of users stored in the server

Paragraph 4. The electronic device 200 of Paragraph 3, wherein the processor 210 may be further configured to obtain, during the designated time interval, trends of acceleration corresponding to each of 3 axis directions; and where the processor 210 may be further configured to identify, based on difference of sliding window summing identified based on the trends of acceleration, the first walking pattern of the user.

Paragraph 5. The electronic device 200 of Paragraph 3 or Paragraph 4, wherein the second information related to the walking environment of the user may include at least one of information on a gradient of a path or route on which the electronic device 200 is moving, information on a moving state of the electronic device 200, and information on weather during the designated time interval.

Paragraph 6. The electronic device 200 of Paragraph 5, wherein the processor 210 may be further configured to obtain the information on the moving state of the electronic device 200 by setting at least of the second data as input data of designated model indicated by a plurality of parameters, stored in the memory 230; and wherein the plurality of parameters of the designated model may be configured based on learning of third data obtained according to the walking history of the user.

Paragraph 7. The electronic device 200 of Paragraph 1 or Paragraph 2, wherein the electronic device 200 may comprise a communication circuit 240; wherein the processor 210 may be configured to receive, using the communication circuit 240, the third information related to the reference walking pattern from a server connected to the electronic device 200; wherein the processor 210 may be further configured to store the third information in the memory 230; and wherein the third information may be obtained based on a plurality of walking patterns related to a plurality of users stored in the server.

Paragraph 8. The electronic device 200 of any of Paragraphs 1 to 7, wherein the processor 210 may be further configured to identify, based on a Euclidean distance between values related to the first walking pattern and values related to the reference walking pattern, the first value; and wherein the processor 210 may be further configured to identify, based on a Euclidean distance between the values related to the first walking pattern and values related to the second walking pattern, the second value.

Paragraph 9. The electronic device 200 of Paragraph 2, wherein the processor 210 may be further configured to perform learning of a walking pattern of the user based on the walking history of the user; wherein the processor 210 may be further configured to, based on performing the learning of the walking pattern of the user, obtain the fourth information related to the second walking pattern; and wherein the processor 210 may be further configured to store the fourth information in the memory 230.

Paragraph 10. The electronic device 200 of Paragraph 9, wherein the processor 210 may be further configured to identify, based on the walking history of the user, a population including values related to a walking pattern according to a plurality of steps of the user; wherein the processor 210 may be further configured to identify at least part of the population based on information on fitness which is obtained based on the population according to the plurality of steps; and wherein the processor 210 may be further configured to perform, based on the at least part of the population, learning of the walking pattern of the user.

Paragraph 11. The electronic device 200 of Paragraph 10, wherein the processor 210 may further configured to identify, using the at least one sensor 220, first steps of the user; wherein the processor 210 may further configured to, based on identifying that the number of the first steps is equal to or greater than a designated number, identify feature values according to the first steps; and wherein the processor 210 may further configured to, based on the feature values according to the first steps, organize the population.

Paragraph 12. The electronic device 200 of Paragraph 11, wherein the processor 210 may be further configured to identify, based on the feature values according to the first steps, values related to a walking pattern according to the first steps; wherein the processor 210 may be further configured to identify, based on feature values according to second steps of the user occurred before the first steps are identified, which are stored in the memory 230, values related to a walking pattern according to the second steps; and wherein the processor 210 may be further configured to organize the values related to a walking pattern according to the first steps and the values related to a walking pattern according to the second steps into the population including values related to the walking pattern according to the plurality of steps.

Paragraph 13. The electronic device 200 of any of Paragraphs 1 to 12, wherein the processor 210 may be further configured to operate one of first mode to fourth mode; wherein the processor 210 may be further configured to, based on the first mode, initialize the walking history of the user and the fourth information related to the second walking pattern which are stored in the memory 230; wherein the processor 210 may be further configured to, based on the second mode, obtain, using the at least one sensor 220, data related to a walking pattern of the user; wherein the processor 210 may be further configured to, based on the third mode, perform learning of data related to the walking pattern of the user; and wherein the processor 210 may be further configured to, based on the fourth mode, cease performing learning of data related to the walking pattern of the user.

Paragraph 14. The electronic device 200 of any of Paragraphs 1 to 13, wherein the processor 210 may be further configured to, based on the first value, the second value, and the second information, identify a probability value for the user to fall; and wherein the processor 210 may be further configured to identify, based on the probability value, one of a plurality of candidate threshold values as the threshold value.

Paragraph 15. The electronic device 200 of Paragraph 14, wherein the processor 210 may be further configured to, based on the probability value and the second information, transmit (e.g., via communication circuit 240 comprised in the electronic device), a signal for causing the external electronic device 2100 to provide a notification to warn of a fall hazard through the external electronic device 2100 connected to the electronic device 200 and worn by the user, to the external electronic device 2100.

Paragraph 16. A method of an electronic device 200 may comprise obtaining, using at least one sensor 220 of the electronic device 200, first information related to a first walking pattern of a user of the electronic device 200 and obtaining second information related to a walking environment of the user; identifying, based on the first information and third information related to a reference walking pattern which is stored in a memory 230 a first value indicating a difference between the first walking pattern and the reference walking pattern; identifying, based on the first information and fourth information related to a second walking pattern of the user, which is stored in the memory 230, a second value indicating difference between the first walking pattern and the second walking pattern; identifying, based on the first value, the second value, and the second information, a threshold value related to fall detection; and executing, based on comparison between the threshold value and a value obtained through the at least one sensor 220, a function for the fall detection.

Paragraph 17. The method of Paragraph 16, wherein the fourth information related to the second walking pattern of the user is identified based on a walking history of the user.

Paragraph 18. The method of Paragraph 17, wherein the method may comprise obtaining, based on first data obtained using an acceleration sensor 221 of the electronic device 200 during a designated time interval, the first information related to the first walking pattern; and wherein the method may comprise obtaining, based on second data obtained using at least one of the acceleration sensor 221, a gyroscope sensor 222 of the electronic device 200, a barometric pressure sensor 223 of the electronic device 200, and a communication circuit 240 of the electronic device 200, the second information related to the walking environment of the user.

Paragraph 19. The method of Paragraph 18, wherein the method may comprise obtaining, during the designated time interval, trends of acceleration corresponding to each of 3 axis directions; and wherein the method may comprise identifying, based on difference of sliding window summing identified based on the trends of acceleration, the first walking pattern of the user.

Paragraph 20. The method of Paragraph 18 or Paragraph 19, wherein the second information related to the walking environment of the user may include at least one of information on a gradient of a moving path of the electronic device 200, information on a moving state of the electronic device 200, and information on weather during the designated time interval.

Paragraph 21. The method of any of Paragraphs 16 to 20, wherein the method may comprise identifying, based on a Euclidean distance between values related to the first walking pattern and values related to the reference walking pattern, the first value and wherein the method may comprise identifying, based on a Euclidean distance between the values related to the first walking pattern and values related to the second walking pattern, the second value.

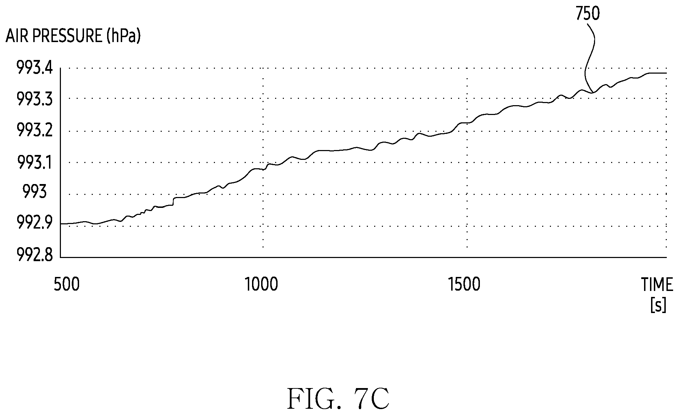

It will be appreciated that, in addition to including the examples, embodiments, aspects etc. disclosed throughout the above, the present disclosure also envisages, and includes, each and every combination of the examples, embodiments, aspects etc. disclosed throughout the above. For instance, each combination of two or more of the examples disclosed herein should be considered to be included in the present disclosure. Furthermore, the present disclosure should also be seen to include examples comprising each and every combination of the various individual features disclosed herein, regardless of whether said individual features belong to the same or different examples, embodiments, aspects etc. To give an example, even without a corresponding literal disclosure, the present disclosure should be seen to comprise examples where any one or more individual features disclosed in combination with FIG. 5 are combined with any one or more individual features disclosed in combination with FIG. 7.

Additionally, it will be appreciated that, for all methods described herein (e.g., including those illustrated in combination with FIGS. 4, 5, 9, 10 and 13), the disclosure also includes versions of each method in which one or more of the described operations are omitted or re-ordered. For example, the disclosure includes versions of each method comprising only one of any of the operations shown in each method, and versions of each method comprising any combination of two or more of the operations shown in each method.

The various examples and terms used herein are not intended to limit the technical features described herein to specific examples and should be understood to include various modifications, equivalents, or substitutes of the example. With respect to the description of the drawings, similar reference numerals may be used for similar or related components. The singular form of the noun corresponding to the item may include one or more of the items unless clearly indicated differently in a related context. In this document, each of the phrases such as "A or B", "at least one of A and B", "A and/or B", "at least one of A or B", "at least one of A, B and C", "at least one of A, B, or C", "A, B and/or C", and "at least one of A, B, or C" may include any one of the phrases together, or all possible combinations thereof. Terms such as "first", "second", or "second", or "second" may be used simply to distinguish a corresponding component from another corresponding component, and are not limited to other aspects (e.g., importance or order). When some (e.g., the first) component is referred to as "coupled" or "connected" in another (e.g., the second) component, with or without the term "functional" or "communicatively", it means that some of the components can be connected directly (e.g., wired), wirelessly, or through a third component.

The term "module" used in various examples of the present document may include a unit implemented in hardware, software, or firmware and be used interchangeably with terms such as logic, logic block, component, or circuitry, for example. The module may be a minimum unit or a part of the integrally configured component or the component that performs one or more functions. For example, according to an example, the module may be implemented in the form of an application-specific integrated circuit (ASIC).

Various examples of the present document may be implemented as software (e.g., a program) including one or more instructions stored in a storage medium (or external memory) readable by a device (e.g., wearable device 100). For example, a processor (e.g., a processor) of a device (e.g., wearable device 100) may call and execute at least one of the one or more instructions stored from a storage medium. This makes it possible for the device to operate to perform at least one function according to at least one command called. The one or more instructions may include code generated by a compiler or code that may be executed by an interpreter. The device-readable storage medium may be provided in the form of a non-transitory storage medium. Here, the term 'non-transitory' only means that a storage medium is a device that is tangible and does not include a signal (e.g., electromagnetic wave), and the term does not distinguish between a case where data is semi-permanently stored and a case where it is temporarily stored.

According to an example, a method according to various examples disclosed in the present document may be provided by being included in a computer program product. The computer program products may be traded between sellers and buyers as products. The computer program products may be distributed in the form of device-readable storage media (e.g., compact disc read only memory (CD-ROM), or distributed (e.g., downloaded or uploaded) directly or online through an application store (e.g., Play Store™) or between two user devices (e.g., smartphones). In the case of online distribution, at least some of the computer program products may be temporarily stored or temporarily created on a device-readable storage medium such as a manufacturer's server, a server in an application store, or a memory in a relay server.

According to various examples, each of the above-described components (e.g., a module or a program) may include a single object or a plurality of objects, and some of the plurality of objects may be separated and disposed in other components. According to various examples, one or more components or operations of the above-described corresponding components may be omitted, or one or more other components or operations may be added. Alternatively, or additionally, a plurality of components (e.g., modules or programs) may be integrated into one component. In this case, the integrated component may perform one or more functions of each of the components in the same or similar manner as those performed by the corresponding component among the plurality of components before the integration. According to various examples, operations performed by a module, a program, or other components may be executed sequentially, in parallel, repeatedly, or heuristic, performed in a different order, omitted, or one or more other operations may be added.

**Claims**

1. An electronic device comprising:

   at least one sensor;
   a memory; and

a processor, wherein the processor configured to:

obtain, using the at least one sensor, first information related to a first walking pattern of a user of the electronic device and obtain second information related to a walking environment of the user;

identify, based on the first information and third information related to a reference walking pattern which is stored in the memory, a first value indicating a difference between the first walking pattern and the reference walking pattern;

identify, based on the first information and fourth information related to a second walking pattern of the user, which is stored in the memory, a second value indicating a difference between the first walking pattern and the second walking pattern;

identify, based on the first value, the second value, and the second information, a threshold value related to fall detection; and

execute, based on comparison between the threshold value and a value obtained through the at least one sensor, a function for the fall detection.

2. The electronic device of claim 1, wherein the fourth information related to the second walking pattern of the user is identified based on a walking history of the user.

3. The electronic device of claim 2, further comprising

a communication circuit,
wherein the at least one sensor includes:

an acceleration sensor;
a gyroscope sensor; and
a barometric pressure sensor,
wherein the processor is further configured to:

obtain, based on first data obtained using the acceleration sensor during a designated time interval, the first information related to the first walking pattern; and

obtain, based on second data obtained using at least one of the acceleration sensor, the gyroscope sensor, the barometric pressure sensor, and the communication circuit, the second information related to the walking environment of the user.

4. The electronic device of claim 3, wherein the processor is further configured to:

obtain, during the designated time interval, trends of acceleration corresponding to each of 3 axis directions; and

identify, based on difference of sliding window summing identified based on the trends of acceleration, the first walking pattern of the user.

5. The electronic device of claim 3 or claim 4, wherein the second information related to the walking environment of the user includes at least one of information on a gradient of a path on which the electronic device is moving, information on a moving state of the electronic device, and information on weather during the designated time interval.

6. The electronic device of claim 5, wherein the processor is further configured to obtain the information on the moving state of the electronic device by setting at least of the second data as input data of designated model indicated by a plurality of parameters, stored in the memory,

wherein the plurality of parameters of the designated model is configured based on learning of third data obtained according to the walking history of the user.

7. The electronic device of claim 1 or claim 2, further comprising a communication circuit,
wherein the processor is further configured to:

receive, using the communication circuit, the third information related to the reference walking pattern from a server connected to the electronic device, and

store the third information in the memory,
wherein the third information is obtained based on a plurality of walking patterns related to a plurality of users stored in the server.

8. The electronic device of one of claim 1 to claim 7, wherein the processor is further configured to:

   identify, based on a Euclidean distance between values related to the first walking pattern and values related to the reference walking pattern, the first value, and
   identify, based on a Euclidean distance between the values related to the first walking pattern and values related to the second walking pattern, the second value.

9. The electronic device of claim 2, wherein the processor is further configured to:

   perform learning of a walking pattern of the user based on the walking history of the user,
   based on performing the learning of the walking pattern of the user, obtain the fourth information related to the second walking pattern, and
   store the fourth information in the memory.

10. The electronic device of claim 9, wherein the processor is further configured to:

    identify, based on the walking history of the user, a population including values related to a walking pattern according to a plurality of steps of the user,
    identify at least part of the population based on information on fitness which is obtained based on the population according to the plurality of steps, and
    perform, based on the at least part of the population, learning of the walking pattern of the user.

11. The electronic device of claim 10, wherein the processor is further configured to:

    identify, using the at least one sensor, first steps of the user,
    based on identifying that the number of the first steps is equal to or greater than a designated number, identify feature values according to the first steps, and
    based on the feature values according to the first steps, organize the population.

12. The electronic device of claim 11, wherein the processor is further configured to:

    identify, based on the feature values according to the first steps, values related to a walking pattern according to the first steps,
    identify, based on feature values related to a walking pattern according to second steps of the user that occurred before the first steps are identified, which are stored in the memory, values related to a walking pattern according to the second steps, and
    organize the population including values related to the walking pattern according to the plurality of steps, based on the values related to a walking pattern according to the first steps and the values related to a walking pattern according to the second steps.

13. The electronic device of claim 1, wherein the processor is further configured to:

    based on the first value, the second value, and the second information, identify a probability value for the user to fall, and
    identify, based on the probability value, one of a plurality of candidate threshold values as the threshold value.

14. The electronic device of claim 13, further comprising a communication circuit,
    wherein the processor is further configured to:
    based on the probability value and the second information, transmit a signal for causing the external electronic device, to provide notification to warn a fall hazard through the external electronic device connected to the electronic device and worn by the user, to the external electronic.

15. A method of an electronic device comprising:

    obtaining, using at least one sensor of the electronic device, first information related to a first walking pattern of a user of the electronic device and obtaining second information related to a walking environment of the user;
    identifying, based on the first information and third information related to a reference walking pattern which is stored in a memory of the electronic device, a first value indicating a difference between the first walking pattern and the reference walking pattern;

identifying, based on the first information and fourth information related to a second walking pattern of the user, which is stored in the memory, a second value indicating difference between the first walking pattern and the second walking pattern;

identifying, based on the first value, the second value, and the second information, a threshold value related to fall detection; and

executing, based on comparison between the threshold value and a value obtained through the at least one sensor, a function for the fall detection.

100

ELECTRONIC DEVICE 101

INPUT MODULE 150

SOUND OUTPUT MODULE 155

DISPLAY MODULE 160

MEMORY 130

VOLATILE MEMORY 132

NON-VOLATILE MEMORY 134

INTERNAL MEMORY 136

EXTERNAL MEMORY 138

BATTERY 189

POWER MANAGE-MENT MODULE 188

PROCESSOR 120

MAIN PROCESSOR 121

AUXILIARY PROCESSOR 123

COMMUNICATION MODULE 190

WIRELESS COMMUNICATION MODULE 192

WIRED COMMUNICATION MODULE 194

SUBSCRIBER IDENTIFICATION MODULE 196

ANTENNA MODULE 197

AUDIO MODULE 170

SENSOR MODULE 176

HAPTIC MODULE 179

CAMERA MODULE 180

INTERFACE 177

CONNECTING TERMINAL 178

PROGRAM 140

APPLICATION 146

MIDDLEWARE 144

OPERATING SYSTEM 142

SECOND NETWORK 199

ELECTRONIC DEVICE 104

FIRST NETWORK 198

ELECTRONIC DEVICE 102

SERVER 108

FIG. 1

FIG. 2

210

310

## FALL DETECTION CIRCUIT

FALL MOTION ANALYSIS CIRCUIT — 311

FALL ENVIRONMENT ANALYSIS CIRCUIT — 312

FALL SENSITIVITY DEGREE ADJUSTMENT CIRCUIT — 313

FALL IDENTIFICATION CIRCUIT — 314

320

## FALL RISK IDENTIFICATION CIRCUIT

WALKING ENVIRONMENT IDENTIFICATION CIRCUIT — 321

WALKING PATTERN LEARNING CIRCUIT — 322

WALKING PATTERN ANALYSIS CIRCUIT — 323

FALL RISK PREDICTION CIRCUIT — 324

330

## FALL NOTIFICATION CIRCUIT

FALL OCCURRENCE NOTIFICATION CIRCUIT — 331

FALL RISK NOTIFICATION CIRCUIT — 332

FIG. 3

```
┌─────────────────────────────────────────┐
│  OBTAIN FIRST INFORMATION RELATED TO FIRST │
│  WALKING PATTERN AND SECOND INFORMATION    │──── 410
│     RELATED TO WALKING ENVIRONMENT         │
└─────────────────────────────────────────┘
                    │
                    ▼
┌─────────────────────────────────────────┐
│  IDENTIFY FIRST VALUE INDICATING DIFFERENCE │
│   BETWEEN FIRST WALKING PATTERN AND        │──── 420
│       REFERENCE WALKING PATTERN            │
└─────────────────────────────────────────┘
                    │
                    ▼
┌─────────────────────────────────────────┐
│      IDENTIFY SECOND VALUE INDICATING       │
│     DIFFERENCE BETWEEN FIRST WALKING       │──── 430
│   PATTERN AND SECOND WALKING PATTERN       │
└─────────────────────────────────────────┘
                    │
                    ▼
┌─────────────────────────────────────────┐
│       IDENTIFY THRESHOLD VALUE RELATED      │
│           TO FALL DETECTION                │──── 440
└─────────────────────────────────────────┘
                    │
                    ▼
┌─────────────────────────────────────────┐
│          EXECUTE FALL DETECTION            │──── 450
└─────────────────────────────────────────┘
```

FIG. 4

IDENTIFY DATA RELATED
TO WALKING OF USER — 510

↓

IDENTIFY WALKING OF USER — 520

↓

IDENTIFY AIR PRESSURE
CHANGE AMOUNT — 530

↓

AIR PRESSURE CHANGE AMOUNT IS LESS THAN FIRST AIR PRESSURE CHANGE AMOUNT? — 540

NO →

AIR PRESSURE CHANGE AMOUNT IS EQUAL TO OR GREATER THAN SECOND AIR PRESSURE CHANGE AMOUNT? — 560

NO →

YES ↓

IDENTIFY THAT USER IS WALKING UPHILL — 550

YES ↓

IDENTIFY THAT USER IS WALKING DOWNHILL — 570

IDENTIFY THAT USER IS WALKING ON FLATLAND — 580

FIG. 5

FIG. 6A

FIG. 6B

FIG. 6C

FIG. 7A

FIG. 7B

AIR PRESSURE (hPa)

750

993.4
993.3
993.2
993.1
993
992.9
992.8

500                 1000                 1500                 TIME
                                                            [s]

FIG. 7C

FIG. 8

FIG. 9

IDENTIFY FIRST STEPS OF USER ~1010

NUMBER OF FIRST STEPS IS EQUAL TO OR GREATER THAN DESIGNATED NUMBER? 1020

NO

YES

IDENTIFY FEATURE VALUES ACCORDING TO FIRST STEPS ~1030

IDENTIFY POPULATION ~1040

FIG. 10

FIG. 11

<u>1210</u>

| STEP | 1 | 2 | 3 | 4 | 5 | 6 | 7 | 8 | 9 | 10 |
|---|---|---|---|---|---|---|---|---|---|---|
| 1 | 0.3822 | -0.307 | -0.266 | -0.5115 | -0.493 | -0.3689 | -1 | -0.3455 | -0.1045 | 0.1218 |
| 2 | 0.3654 | -0.5648 | -0.3287 | -0.5597 | -0.5101 | -0.5198 | -1 | -0.2555 | -0.189 | 0.2068 |
| 3 | 0.147 | -0.487 | -0.3197 | -0.2887 | -0.2683 | -0.3482 | -0.6 | -0.2903 | -0.006341 | 0.181 |
| 4 | 0.4113 | -0.3658 | -0.1267 | -0.6626 | -0.6686 | -0.4219 | -0.2 | -0.3105 | -0.1241 | 0.2091 |
| 5 | 0.5039 | -0.7255 | -0.4715 | -0.808 | -0.5771 | -0.6979 | -0.6 | -0.1024 | -0.01424 | 0.1858 |
| 6 | 0.4446 | -0.6323 | -0.2566 | -0.7815 | -0.7347 | -0.6065 | -0.6 | -0.1616 | -0.03346 | 0.127 |
| 7 | 0.4738 | -0.08193 | 0.09527 | -0.2206 | -0.2089 | -0.2806 | -0.2 | -0.1567 | -0.4325 | 0.5338 |
| 8 | 0.2976 | -0.1682 | 0.1052 | -0.2623 | -0.2454 | -0.2348 | -0.6 | -0.08752 | -0.2164 | 0.5188 |
| 9 | 0.1597 | -0.1501 | -0.04781 | -0.08824 | -0.1049 | -0.1457 | -0.6 | -0.09516 | -0.4143 | 0.5435 |
| 10 | 0.325 | -0.1489 | 0.01849 | -0.1824 | -0.1889 | -0.2383 | -0.6 | -0.1992 | -0.5517 | 0.5837 |

FEATURE VALUE

FIG. 12

FIG. 13

<u>1410</u>

| | 1 | 2 | 3 |
|---|---|---|---|
| 1 | -0.4186 | 0.1537 | 0.333 |
| 2 | 0.3213 | 0.1872 | 0.1004 |
| 3 | -0.0008575 | 0.008313 | -0.01091 |
| 4 | 0.4335 | -0.02048 | -0.1225 |
| 5 | 0.4127 | -0.01658 | -0.08431 |
| 6 | 0.4362 | 0.02972 | -0.1255 |
| 7 | -0.2309 | -0.7579 | -0.36 |
| 8 | 0.1816 | -0.3695 | 0.8408 |
| 9 | 0.2237 | -0.04373 | 0.004698 |
| 10 | 0.1945 | -0.4763 | 0.06724 |

FIG. 14

1510

| | 1 | 2 | 3 |
|---|---|---|---|
| 1 | -0.6911 | -0.5685 | -0.357 |
| 2 | -0.6822 | -0.5255 | -0.3628 |
| 3 | -0.8856 | -0.5699 | -0.3583 |
| 4 | -0.6571 | -0.5918 | -0.2931 |
| 5 | -0.7281 | -0.5664 | -0.2981 |
| 6 | -0.701 | -0.5713 | -0.3127 |
| 7 | -0.7084 | -0.5654 | -0.3641 |
| 8 | -0.5919 | -0.5562 | -0.3195 |
| 9 | -0.5207 | -0.5759 | -0.3056 |
| 10 | -0.9074 | -0.5553 | -0.3278 |
| 11 | -0.8164 | -0.5666 | -0.2919 |
| 12 | -0.8289 | -0.5525 | -0.2865 |
| 13 | -1.021 | -0.5429 | -0.3219 |
| 14 | -0.5505 | -0.4607 | -0.3209 |
| 15 | -0.6827 | -0.4752 | -0.3182 |
| 16 | -0.782 | -0.4843 | -0.3132 |
| 17 | -0.7866 | -0.5159 | -0.2906 |
| 18 | -0.7385 | -0.5503 | -0.2201 |
| 19 | -0.824 | -0.4696 | -0.3026 |
| 20 | -0.7805 | -0.4646 | -0.3178 |

VALID WALKING INTERVAL

1511 (rows 1–10)

1512 (rows 11–20)

FIG. 15

<u>1610</u>

| | |
|---|---|
| 1 | 0.07545 |
| 2 | 0.07959 |
| 3 | 0.1518 |
| 4 | 0.1054 |
| 5 | 0.03761 |
| 6 | 0.05555 |
| 7 | 0.0679 |
| 8 | 0.1537 |
| 9 | 0.2272 |
| 10 | 0.1648 |
| 11 | 0.08131 |
| 12 | 0.0905 |
| 13 | 0.2771 |
| 14 | 0.2082 |
| 15 | 0.08693 |
| 16 | 0.06436 |
| 17 | 0.05268 |
| 18 | 0.09527 |
| 19 | 0.1047 |
| 20 | 0.0806 |

FIG. 16

1710

| | 1 | 2 | 3 |
|---|---|---|---|
| 1 | -1.021 | -0.5429 | -0.3219 |
| 2 | -0.5207 | -0.5759 | -0.3056 |
| 3 | -0.5505 | -0.4607 | -0.3209 |
| 4 | -0.9074 | -0.5553 | -0.3278 |
| 5 | -0.5919 | -0.5562 | -0.3195 |
| 6 | -0.8856 | -0.5699 | -0.3583 |
| 7 | -0.6571 | -0.5918 | -0.2931 |
| 8 | -0.824 | -0.4696 | -0.3026 |
| 9 | -0.7385 | -0.5503 | -0.2201 |
| 10 | -0.8289 | -0.5525 | -0.2865 |

FIG. 17

FIG. 18

FIG. 19

FIG. 20A

FIG. 20B

FIG. 21A

FIG. 21B

## INTERNATIONAL SEARCH REPORT

| International application No. |
|---|
| **PCT/KR2023/009120** |

| A. | CLASSIFICATION OF SUBJECT MATTER |
|---|---|

G08B 21/04(2006.01)i; G01P 15/00(2006.01)i; G01L 19/08(2006.01)i; G01D 21/02(2006.01)i; G06N 20/00(2019.01)i

According to International Patent Classification (IPC) or to both national classification and IPC

| B. | FIELDS SEARCHED |
|---|---|

Minimum documentation searched (classification system followed by classification symbols)

G08B 21/04(2006.01); A43B 17/00(2006.01); A43B 3/00(2006.01); A61B 5/11(2006.01); G01P 15/00(2006.01); G06N 3/08(2006.01); G08B 21/02(2006.01); G08B 25/10(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 보행(walking), 패턴(pattern), 환경(environment), 정보(information), 낙상(fall), 감지(detect), 임계(threshold), 비교(comparison)

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | KR 10-2008-0050993 A (ELECTRONICS AND TELECOMMUNICATIONS RESEARCH INSTITUTE) 10 June 2008 (2008-06-10)<br>See paragraphs [0010]-[0058]; and figure 1. | 1-7,9-12,15 |
| Y | KR 10-2374021 B1 (GWANGJU INSTITUTE OF SCIENCE AND TECHNOLOGY) 15 March 2022 (2022-03-15)<br>See paragraphs [0007]-[0018]. | 1-7,9-12,15 |
| Y | KR 10-2022-0061473 A (GILON, INC.) 13 May 2022 (2022-05-13)<br>See paragraphs [0027] and [0108]-[0114]. | 10-12 |
| A | KR 10-2019-0056132 A (LG ELECTRONICS INC.) 24 May 2019 (2019-05-24)<br>See paragraphs [0051] and [0131]-[0143]; and figure 2. | 1-7,9-12,15 |
| A | US 2013-0120147 A1 (VITAL CONNECT, INC.) 16 May 2013 (2013-05-16)<br>See paragraphs [0027] and [0039]; and figure 3. | 1-7,9-12,15 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **19 October 2023** | **19 October 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2023/009120**

| Box No. II | Observations where certain claims were found unsearchable (Continuation of item 2 of first sheet) |
|---|---|

This international search report has not been established in respect of certain claims under Article 17(2)(a) for the following reasons:

1. ☐    Claims Nos.:
   because they relate to subject matter not required to be searched by this Authority, namely:

2. ☑    Claims Nos.: **14**
   because they relate to parts of the international application that do not comply with the prescribed requirements to such an extent that no meaningful international search can be carried out, specifically:

   Claim 14 refers to a claim violating the manner of referring to multiple dependent claims (PCT Rule 6.4(a)), and thus is unclear.

3. ☑    Claims Nos.: **8,13**
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/KR2023/009120**

| Patent document cited in search report | | | Publication date (day/month/year) | Patent family member(s) | | | Publication date (day/month/year) |
|---|---|---|---|---|---|---|---|
| KR | 10-2008-0050993 | A | 10 June 2008 | KR | 10-0873495 | B1 | 15 December 2008 |
| | | | | US | 2008-0133277 | A1 | 05 June 2008 |
| | | | | US | 7961109 | B2 | 14 June 2011 |
| KR | 10-2374021 | B1 | 15 March 2022 | KR | 10-2021-0136323 | A | 17 November 2021 |
| KR | 10-2022-0061473 | A | 13 May 2022 | KR | 10-2421699 | B1 | 18 July 2022 |
| | | | | WO | 2022-097795 | A1 | 12 May 2022 |
| KR | 10-2019-0056132 | A | 24 May 2019 | WO | 2019-098582 | A1 | 23 May 2019 |
| US | 2013-0120147 | A1 | 16 May 2013 | US | 2013-0120152 | A1 | 16 May 2013 |
| | | | | US | 8614630 | B2 | 24 December 2013 |
| | | | | US | 9588135 | B1 | 07 March 2017 |
| | | | | US | 9818281 | B2 | 14 November 2017 |
| | | | | WO | 2013-074538 | A1 | 23 May 2013 |

Form PCT/ISA/210 (patent family annex) (July 2022)